# EUROPEAN PATENT APPLICATION

(11) **EP 0 541 966 A2**
(43) Date of publication of application: **19.05.1993**
(21) Application number: 92117375.3
(22) Date of filing: 12.10.1992
(51) Int. Cl.: C07D 251/66, C07D 251/70, C07D 401/14, C07D 403/14, C07D 251/48, C07B 43/06, C07B 43/10

(54) **Process for preparing amide derivatives from halomines and acid halides**

(30) Priority: 15.11.1991 US 793077
(71) Applicant: AMERICAN CYANAMID COMPANY, Stamford Connecticut 06904-0060 (US)
(72) Inventor: Gupta, Ram B., Bronx, New York 10458 (US)
(74) Representative: DIEHL GLAESER HILTL & PARTNER

(57) **Abstract**

This invention provides a process for preparing amide derivatives of acids by the reaction of haloamines and acid halides.

Melamine derived acid amides are prepared by reaction of trichloro and hexachloromelamines with chloroformates and acid chlorides. The by-product chlorine may be recycled in this process.

Amides, carbamates, sulfonamides, phosphoramides, and related amide derivatives may be prepared by the novel process of the invention.

## Description

### FIELD OF THE INVENTION

This invention relates to the preparation of amide derivatives of acids by a novel reaction which combines the amino group of a haloamine with the non-halide portion of an acid halide. The reaction also combines the halogen atom of said haloamine with the halogen atom of said acid halide producing a halogen molecule as a by-product.

### BACKGROUND OF THE INVENTION

Amides can be prepared by the reaction of amines with acid halides. Although the reaction of amines with acid chlorides is quite general, it is sluggish and often fails to take place if the amine is deactivated by the presence of at least one electron withdrawing substituent. In some cases, preparation of an amide may be possible by first deprotonating the amine with a strong base to generate an anion and then allowing the anion to react with the acid halide. This approach, however, is often impractical, costly, and very limited in scope, and is inoperative if the amine has low solubility, high molecular weight, or both.

Melamine chemistry, particularly halomelamine chemistry including preparation and applications, is discussed in an article by B. Bann and S. A. Miller entitled "Melamine and Derivatives of Melamine", Chemical Reviews, Volume 58, pages 131 to 172, (1958). It is stated therein on page 148 that acyl halides such as benzoyl chloride in the Schotten-Baumann reaction have no effect on melamine. The inertness of melamine towards acid halides is further described in Chemical Abstracts Vol. 30, P. 465 (1936) and in U.S. Pat. No. 2,577,418.

Partially successful attempts to prepare mono- and bis-carbamates from aminotriazines and haloformates are described in an article entitled "Melamine Derivatives 18: Reactions of Melamine and Benzoguanamine with Ethyl Chlorocarbonate" by H. Kitajima, T. Imanaka, and T. Yamomoto in Yuki Gosei Kagaku Kyokai Shi, Volume 32, Number 9, pages 723 to 726 (1974); Chemical Abstracts Volume 82, Number 11: 72946d. The article, however, does not mention the preparation of tris-carbamates.

Tris-carbamates have been prepared by the generally inefficient and costly method of converting an aminotriazine to an isocyanate group followed by reaction with an alcohol. This approach is described in U.S. Pat. No. 4,939,213. The patent also describes curable compositions using triazine tris-carbamates. The preparation of very few other carbamates by the abovementioned approach is described in an article by U. Von Gizycki in Angewandte Chemie, International Edition, Volume 10, page 403, (1971), entitled "Isocyanato-s-Triazines." The author states therein that until that time, only one isocyanato-s-triazine, namely the 2,4-dichloro-6-isocyanate derivative had previously been isolated, and that from tetrameric cyanogen chloride by a route that cannot be generalized.

Preparation of halomelamines are disclosed in U.S. Pat. Nos. 2,184,888; 2,184,886; 2,184,883; 3,743,642; and 2,472,361; in South African Pat. No. 66-03546; and in European Pat. No. 239,121. The reaction of N,N-dichloroamines and N,N-dichloroamides with oxalyl chloride is disclosed in Chemical Abstracts, Vol. 75 (21), item 129306g, condensed from a Russian Language article in Zh. Org. Khim., Vol. 7, No. 7, P. 1541 (1971). The reaction of N-chlorocarboximidic esters with oxalyl chloride is also disclosed in Chemical Abstracts, Vol. 72 (17), item 90006v, condensed from a Russian Language article in Zh. Org. Khim., Vol. 6, No. 1, p. 85-88 (1970).

In view of the reported difficulties encountered by practitioners in the fields of melamine and triazine chemistry, a novel chemical reaction which overcomes the aforementioned difficulties and provides a general method for preparing acylated melamines from widely available halomelamine precursors would be a valuable addition to the very limited methods available to date. When not commerically available, the haloamines are easily prepared by well known methods.

It is the object of this invention to provide a simple process for preparing amides from haloamines and acid halides.

The novel approach of the invention involves a novel chemical reaction which combines the amine group of the haloamine with the non-halide portion of an acid halide to produce an acid amide and recyclable halogen.

### SUMMARY OF THE INVENTION

This invention provides a process for preparing amide derivatives of acids from haloamines and acid halides.

The amides are prepared by a novel reaction which combines the amino group of the haloamine with the non-halide portion of the acid halide. Concurrently, the novel reaction also combines the halogen atom of said haloamine with the halogen atom of said acid halide producing a halogen molecule as a recyclable by-product.

The process of the invention has many advantages some of which are described hereinbelow. For example, the process for the invention affords products which are characterized by low salt, low color, high purity, and high yield. The process is also applicable for use with acid or base sensitive substrates, and is carried out in a neutral medium. Most importantly, it allows facile functionalization and isolation of aminotriazine derivatives such as melamine and benzoguanamine, and related amino compounds such as glycoluril and urea, without the use of formaldehyde. A final advantage of the process described herein is that it allows the facile functionalization of insoluble amines such as melamine by a simple halogenation, acylation, and dehalogenation cycle.

This invention also provides novel products prepared by the process of the invention.

### BRIEF DESCRIPTION OF THE DRAWINGS

FIGURE 1 schematically illustrates a process for producing triazine tris-methyl carbamate from melamine via trichloromelamine.

FIGURE 2 schematically illustrates a process for producing triazine tris-methyl carbamate from malamine via hexachloromelamine.

### DETAILED DESCRIPTION

This invention is a novel process for preparing an acid amide from a haloamine and an acid halide comprising the step of contacting said haloamine with said acid halide at a temperature and for a length of time sufficient to produce said acid amide as a product and halogen as a by-product.

The term "acid halide" herein refers to a derivative of weak or strong hydroxylic acids wherein the hydroxy group is converted to a halogen. An example of the acid halides of the invention is acetyl chloride which may be derived from acetic acid by converting the hydroxy group to chloride by a suitable reagent. Further examples of the acid halides of the invention are listed below: 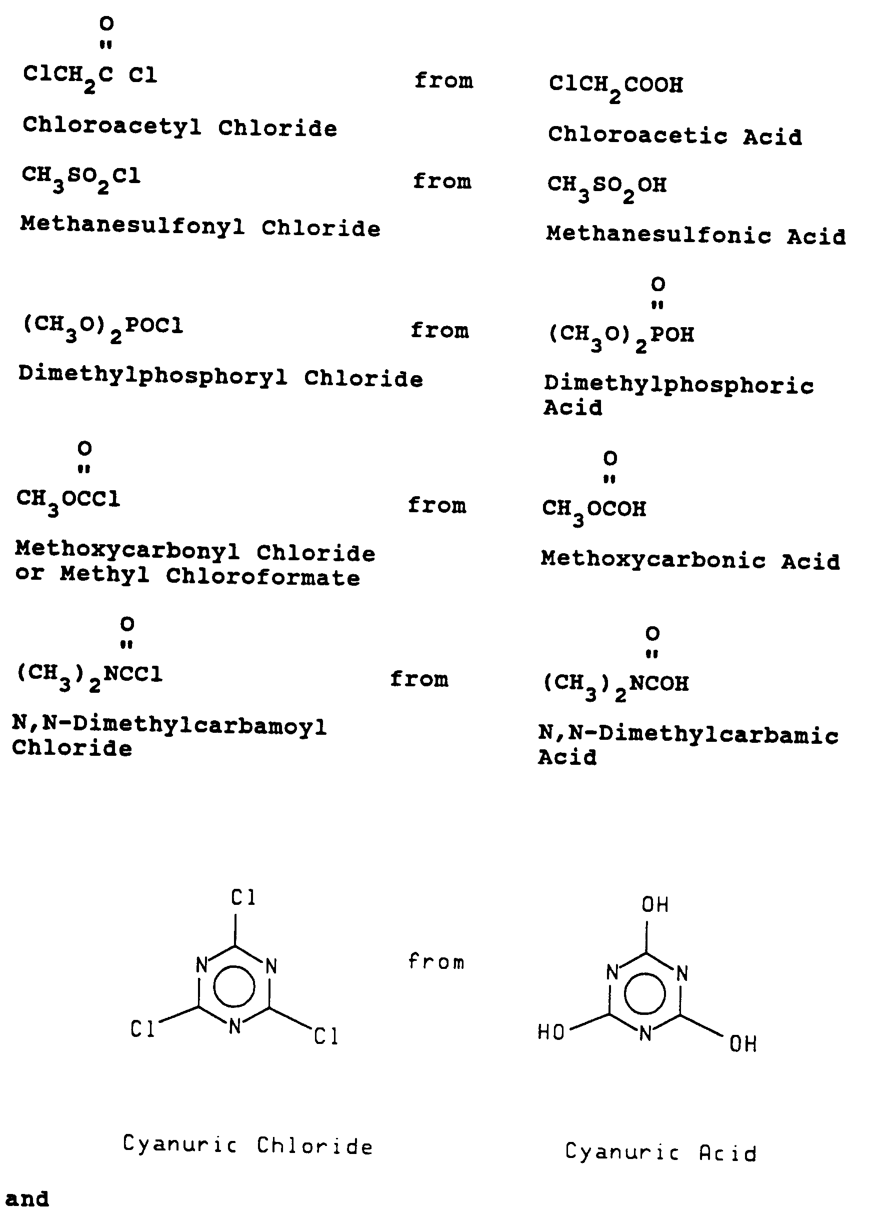 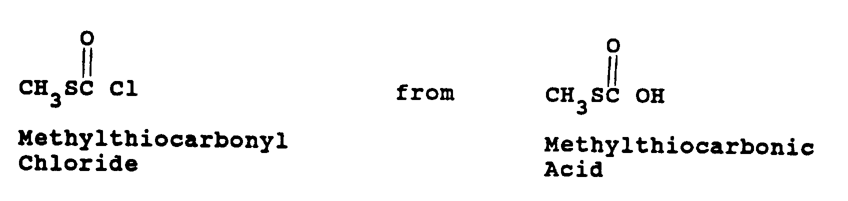
The acid halides examplified above and other halides which fulfill the above requirements are within the meaning of the term "acid halide" as used in the context of this invention.

The term "acid amide" is used in the context of this invention to denote products derived from acid halides by a simple replacement of the halide atoms thereof with an amino group. Furthermore, in the context of this invention, a carbamate may be viewed to be an "acid amide" as defined herein, because a carbamate may be derived from a halo-formate by replacement of the halide with an amino group.

The term "haloamine" is defined herein as an amine compound in which at least one nitrogen-bound hydrogen is replaced by a halogen atom.

The acid halide and haloamine reactants and the acid amides resulting from the novel reaction thereof are described in greater detail below.

### THE NOVEL REACTION

The reaction of amines with acid halides is known and may be examplified by Equation (1): 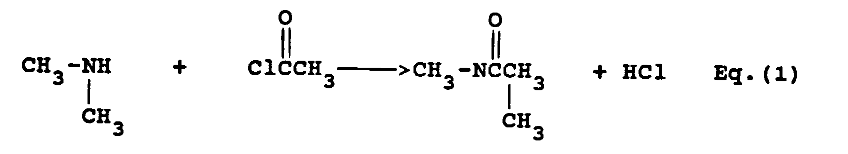
The analogous reaction of a haloamine with an acid halide would be expected to take place according to Equation (2): 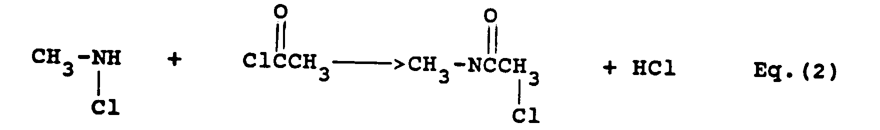
We have surprisingly found that, instead of the expected reaction depicted in Equation (2), a novel, heitherto unreported reaction takes place between a haloamine and an acid halide. The novel reaction is examplified by Equation (3): 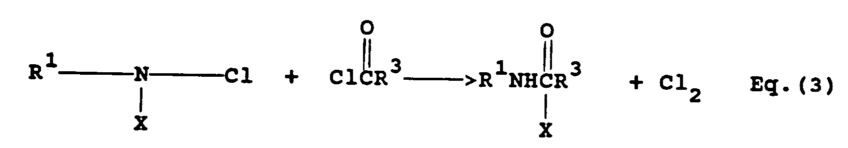
wherein X is R², hydrogen, or halogen, and wherein R¹ and R² are substituents on the haloamine nitrogen and R³ is a substituent on the acid halide.

Reactions according to Equation (3) and other analogous amide-forming reactions of haloamines and acid halides is referred to herein as "the novel reaction of the invention."

### THE HALOAMINES

The haloamines usable in this invention are amine compounds wherein at least one nitrogen bound hydrogen is replaced by a halogen atom.

The amine precursors of the haloamines of the invention may be monomeric or polymeric. They may be very soluble or sparingly soluble in organic solvents. The most particularly useful haloamines are those haloamines which are derived from amine precursors that have solubilities less than 10 weight percent in organic solvents, particularly in halogenated organic solvents.

The haloamines usable in the process of this invention may be open chain or cyclic. The open chain haloamines may be represented by the formula: 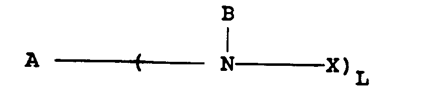
wherein
- L: is at least 1; and when L is at least 2, each 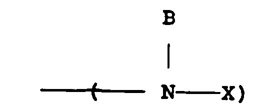 group is the same or different;
- X: is a halogen selected from the group consisting of chloro, bromo, iodo, fluoro, and mixtures thereof;
- B: is selected from the group consisting of hydrogen, chloro, bromo, iodo, fluoro, aminocarbonyl, alkylaminocarbonyl of 2 to 20 carbon atoms, dialkylaminocarbonyl of 3 to 40 carbon atoms, heteroaromatic ring, alkylthiocarbonyl, silyl, cyano, perfluoroalkyl, perfluoroaryl, perfluoroaralkyl, and mixtures thereof;
- A: is an L-functional anchor selected from the group consisting of monomeric organic radical, polymeric organic radical, hydrogen, chloro, bromo, iodo, fluoro, aminocarbonyl, alkylaminocarbonyl of 2 to 20 carbon atoms, dialkylaminocarbonyl of 3 to 40 carbon atoms, heteroaromatic ring, alkylthiocarbonyl, silyl, cyano, perfluoroalkyl, perfluoroaryl, perfluoroaralkyl, phthaloyl, succionyl, maleoyl, orthophenylenedisulfonyl, 1,2-ethylenedisulfonyl, anchors represented by the following formulae:

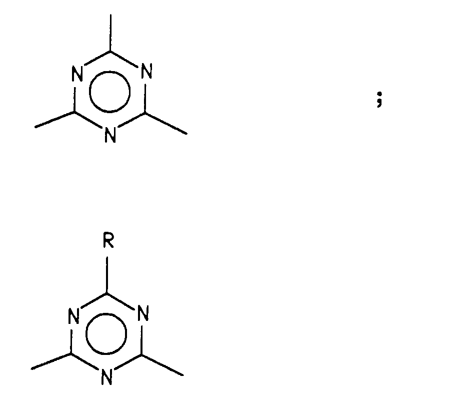

wherein
- R: is selected from the group consisting of alkyl of 1 to 20 carbon atoms, alkenyl of 2 to 20 carbon atoms, aryl of 6 to 20 carbon atoms, aralkyl of 7 to 20 carbon atoms, alkoxy of 1 to 20 carbon atoms, aryloxy of 6 to 20 carbon atoms, alkylthio of 1 to 20 carbon atoms, arylthio of 6 to 20 carbon atoms, alkylamino of 1 to 20 carbon atoms, dialkylamino of 2 to 40 carbon atoms, morpholino, piperidino, pyrrolidino, hydrogen, chloro, bromo, iodo, fluoro, perfluoroalkyl, perfluoroaryl, and perfluoroaralkyl groups;

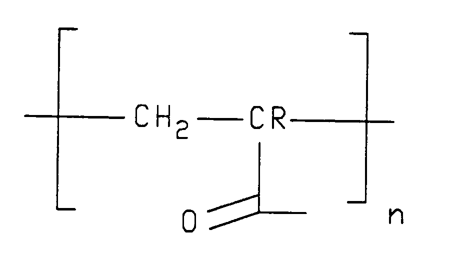

wherein
- R: is hydrogen, alkyl, or aryl; and
- n: is at least 1; and

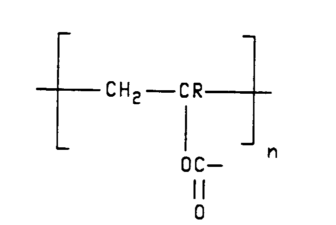

wherein
- R: is hydrogen, alkyl, or aryl; and
- n: is at least 1, and mixtures thereof.

The preferred halogen in the haloamine is chloride and the preferred haloamines are monochloromelamine, dichloromelamine, trichloromelamine, tetrachloromelamine, pentachloromelamine, hexachloromelamine, isomers thereof, and mixtures of at least two of any of the abovementioned chloromelamines.

The chloromelamines described above are represented by the formulae: 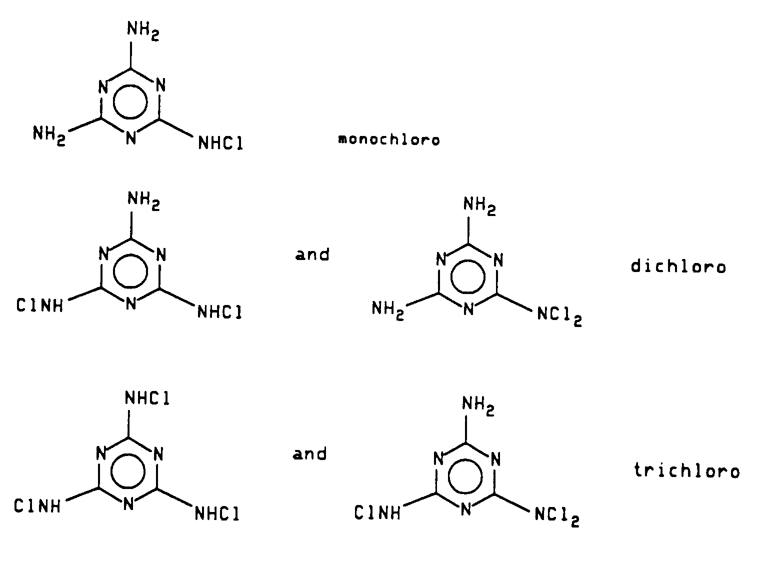 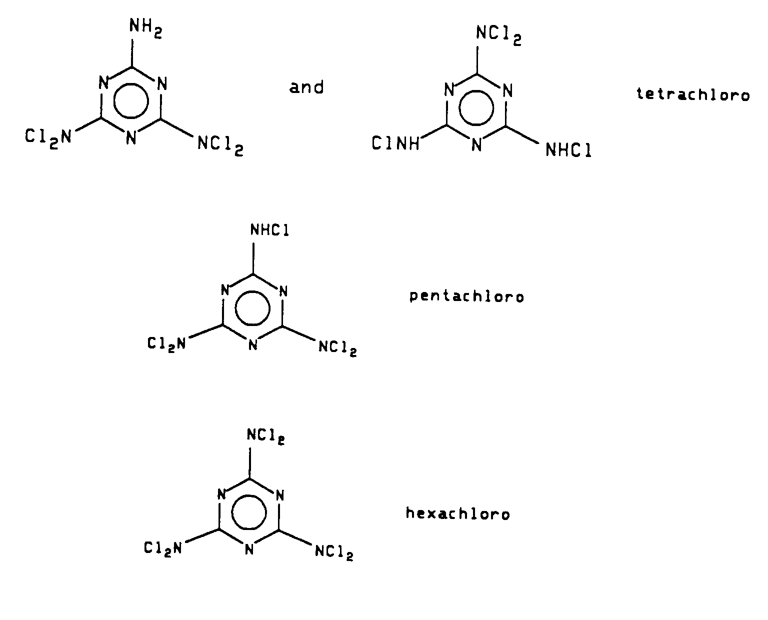
The most preferred chloromelamines are hexachloromelamine and N,N',N''-trichloromelamine isomers represented by the formulae:

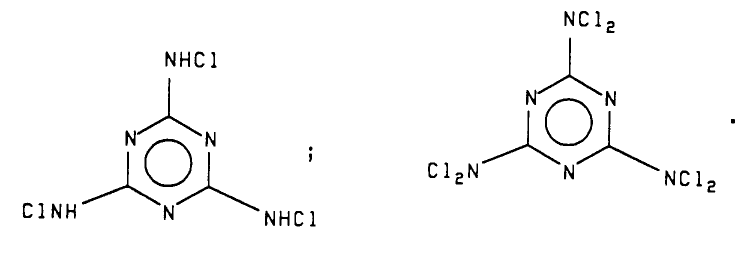

N,N',N''-Trichloromelamine (also known as N,N',N''-trichloro-2,4,6-triamino-1,3,5-triazine) is commerically available from Aldrich Chemical Company, Milwaukee, WI, as an off white powder. The preparation of mono-, di-, tri-, and hexa-chloromelamine, and bromo- and iodo-melamines is reviewed in pages 159 to 162 of the article by B. Bann and S. A. Miller cited previously hereinabove and is further described in U.S. Pat. No. 2,472,361 in detail.

Haloguanamines are also among the preferred haloamines of the invention and include N-substituted mono-, di-,tri-, and tetra- halo acetoguanamines and benzoguanamines.

Cyclic haloamines are also suitable for use in the practice of this invention and may be viewed to arise by connecting groups A and B of the open chain haloamines with a chemical bond.

The cyclic haloamines may be represented by the formula: 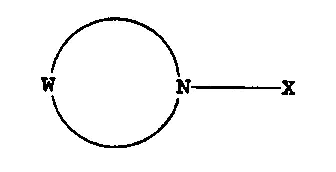
wherein W is a difunctional group capable of forming a ring at least three membered in size, and preferably larger.

The difunctional group W is selected from the group consisting of phthaloyl, succinoyl, maleoyl, alkylene, arylene, 2,2-diphenylene, aralkylene, orthophenylenedisul- fonyl, 1,2-ethylene disulfonyl, a difunctional group represented by the formula: 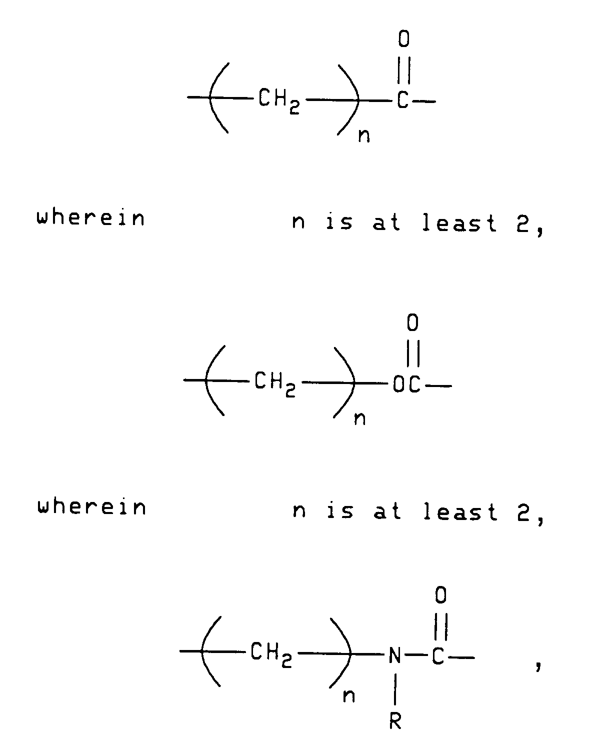
wherein
- n: is at least 2, and
- R: is selected from the group consisting of hydrogen, halogen, alkyl of 1 to 20 carbon atoms, aryl of 6 to 20 carbon atoms; and aralkyl of 7 to 20 carbon atoms; and
ortho-sulfobenzoyl group represented by the formula: 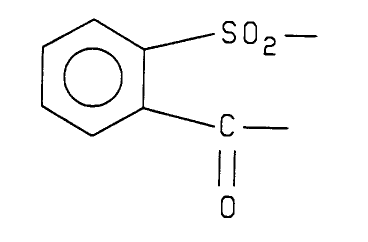
N-Haloglycolurils, which are a special class of cyclic haloamines, are also usable in the process of the invention. The N-haloglycolurils are represented by the formula: 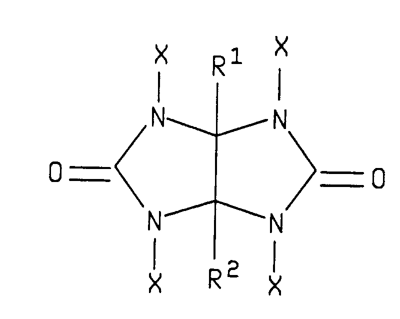
wherein
R¹ and R² are the same or different and are selected from the group consisting of hydrogen, alkyl of 1 to 20 carbon atoms, aryl of 6 to 20 carbon atoms, and aralkyl of 7 to 20 carbon atoms; and
wherein
X is halogen selected from the group consisting of chloride, bromide, iodide, fluoride, and mixtures thereof.
The preferred glycoluril derivative is N,N',N'',N'''-tetrachloroglycoluril represented by the formula:

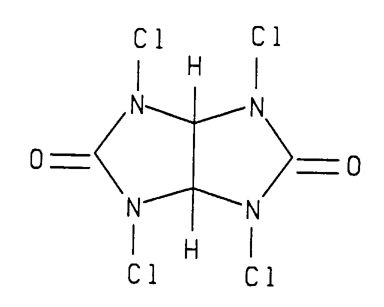

### THE ACID HALIDES

The acid halides usable in the practice of this invention may be represented generically by the formula:

Z(̵Y-X)_{M}

wherein
- M: is at least 1; and when M is at least 2, each (̵Y-X) group is the same or different;
- X: is a halogen selected from the group consisting of chloride, bromide, iodide, fluoride, and mixtures thereof;
- Y: in each (̵Y-X) group is the same or different and each is selected independently from the group consisting of the following functionalities represented by the formulae: 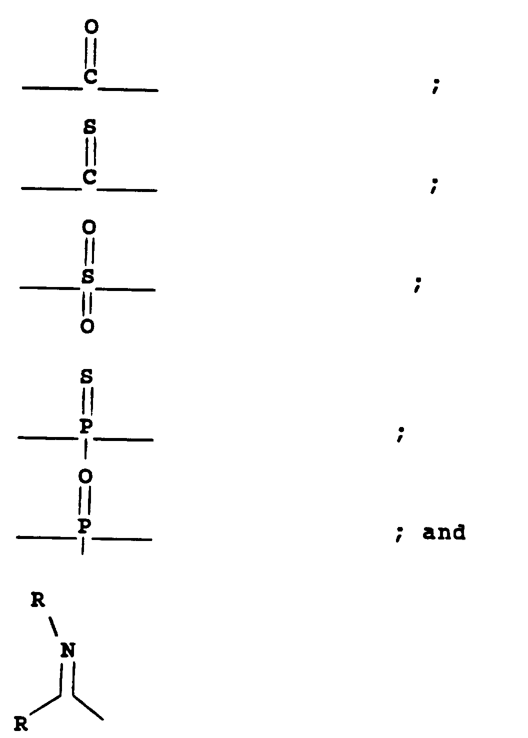 wherein each R is independently an alkyl, aryl, or an alkoxy group; and
- Z: is an M-functional anchor selected from the group consisting of monomeric or polymeric organic radical, hydrogen, chloro, bromo, iodo, fluoro, vinyl, alkyl of 1 to 20 carbon atoms, haloalkyl of 1 to 20 carbon atoms, aryl of 6 to 20 carbon atoms, haloaryl of 6 to 20 carbon atoms, aralkyl of 7 to 20 carbon atoms, haloaralkyl of 7 to 20 carbon atoms, acyl of 2 to 20 carbon atoms, haloacyl of 2 to 20 carbon atoms, halocarbonyl, alkoxycarbonyl of 2 to 20 carbon atoms, alkylthiocarbonyl of 2 to 20 carbon atoms, haloalkoxycarbonyl of 2 to 20 carbon atoms, aminocarbonyl, alkylaminocarbonyl of 2 to 20 carbon atoms, dialkylaminocarbonyl of 3 to 20 carbon atoms, alkenylaminocarbonyl of 4 to 20 carbon atoms, dialkenylaminocarbonyl of 7 to 40 carbon atoms, alkylalkenylaminocarbonyl of 5 to 20 carbon atoms, heteroaromatic ring, perfluoroalkyl of 1 to 20 carbon atoms, perfluoroaryl of 6 to 20 carbon atoms, perfluoro aralkyl of 7 to 20 carbon atoms, haloalkyl of 1 to 20 carbon atoms, alkoxycarbonylalkyl of 3 to 20 carbon atoms, cyanoalkyl of 2 to 20 carbon atoms, formylalkyl of 2 to 20 carbon atoms, ketoalkyl of 3 to 20 carbon atoms, trialkoxy- silylalkyl of 4 to 20 carbon atoms, dialkylaminoalkyl of 3 to 20 carbon atoms, alkoxyalkyl of 2 to 20 carbon atoms, alkoxy of 1 to 20 carbon atoms, aryloxy of 6 to 20 carbon atoms, alkenyloxy of 3 to 20 carbon atoms, N,N-dialkylamino of 2 to 40 carbon atoms, N,N-dialkenylamino of 6 to 40 carbon atoms, N,N-diarylamino of 12 to 40 carbon atoms, N,N-alkylalkenylamino of 4 to 20 carbon atoms, N,N-alkylarylamino of 7 to 20 carbon atoms, N,N-alkenylarylamino of 9 to 20 carbon atoms, aziridino, azetidino, pyrrolidino, piperidino, morpholino, alkylmercapto of 1 to 20 carbon atoms, alkenylmercapto of 3 to 20 carbon atoms, and arylmercapto of 6 to 20 carbon atoms.

The preferred halogen is chloride, the preferred Y group is a carbonyl group, and the preferred anchor Z group is selected from alkyl, haloalkyl, and alkoxy groups.

Among the preferred acid halides suitable for use in the practice of the invention are the following classes:
alkyl chloroformates
aryl chlorofomates
acyl chlorides
haloalkylcarbonyl chlorides
acryloyl chlorides
alkyloxallyl chlorides
carbamoyl chlorides, and
phosgene.

Specific examples of acid halides usable in this invention are the following compounds represented by the formulae:

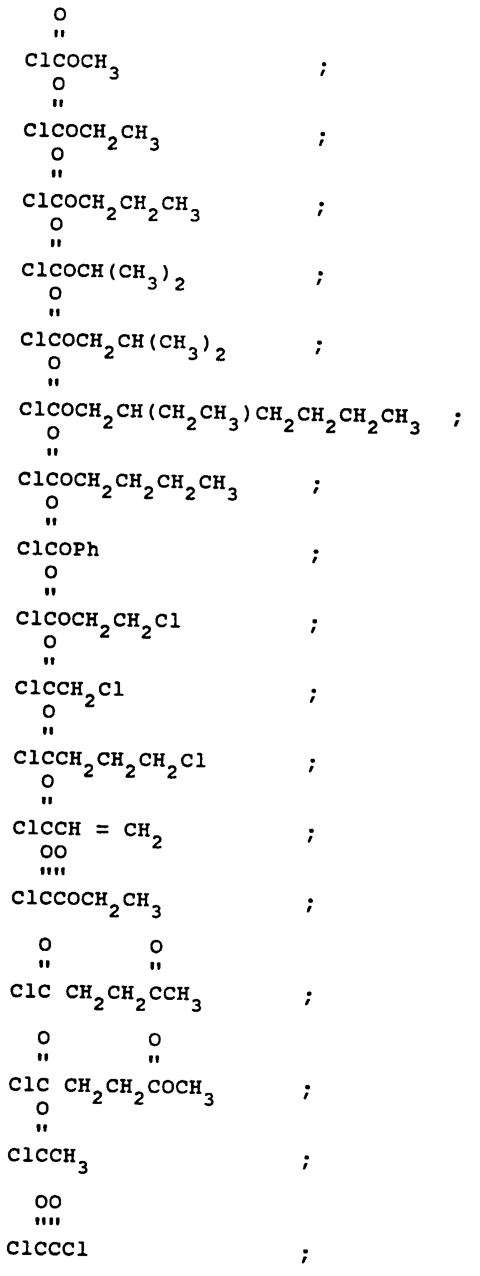

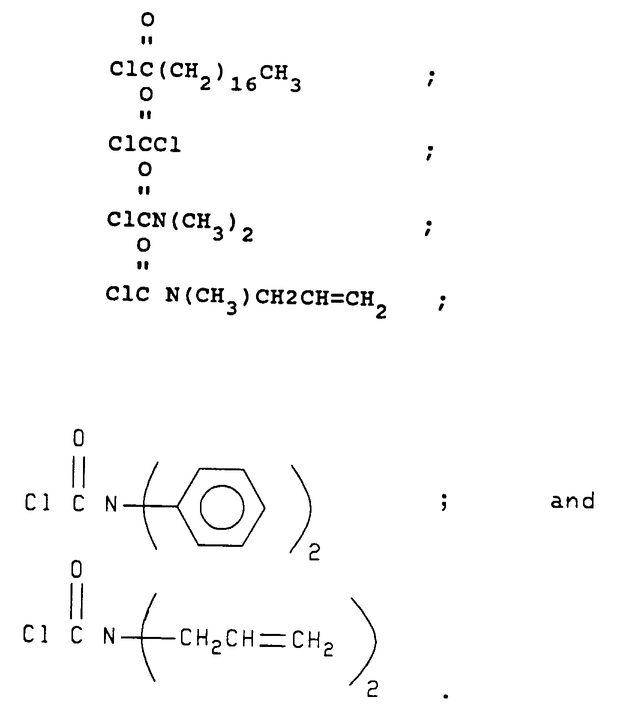

The most preferred acid halides are methyl chloroformate, n-butyl chloroformate, phenyl chloroformate, 2-chloroethyl chloroformate, ethyl chloroformate, propyl chloroformate, isopropyl chloroformate, isobutyl chlorformate, 2-ethylhexyl chloroformate, chloroacetyl chloride, 4-chlorobutyryl chloride, acryloyl chloride, methacryloyl chloride, oxalyl chloride, ethyloxalyl chloride, benzoyl chloride, para-nitrobenzoyl chloride, levulinic chloride, methyl fumaroyl chloride, acetyl chloride, stearoyl chloride, and phosgene.

### THE ACID AMIDE PRODUCTS

The acid amide products resulting from the reaction of open chain haloamines and acid halides described above are represented, when M=1, by the formula: 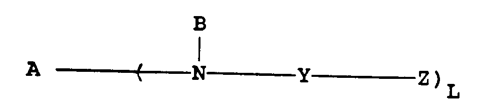
wherein A, B, Y, Z, and L are as defined previously above.

In cases where M is greater than 1 but L is equal to 1, the acid amide products of the invention may be represented by the formula: 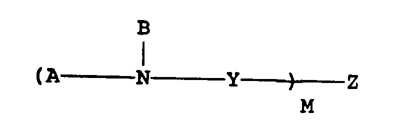
wherein A, B, Y, Z, and M are as defined previously above.

In cases where both L and M are equal to 2, a linear amide oligomer or polymer may be formed.

Lastly, in cases where one of either L or M is at least 2, and the other one is greater than 2, a crosslinked polyamide network may be formed.

The acid amide products resulting from the reaction of cyclic haloamines and the acid halides may be represented by the formula: 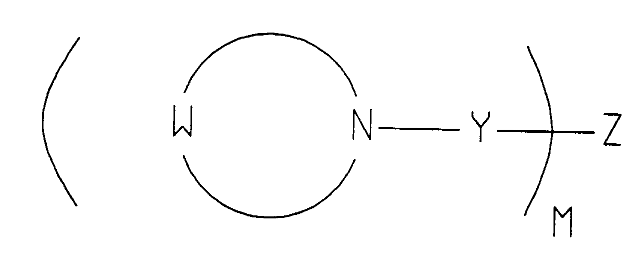
wherein W, Y, Z, and M are as defined previously above.

An example of the acid amide products of the invention resulting from the reaction of N,N',N''-trichloromelamine with methyl chloroformate is the triazine tris-carbamate represented by the formula: 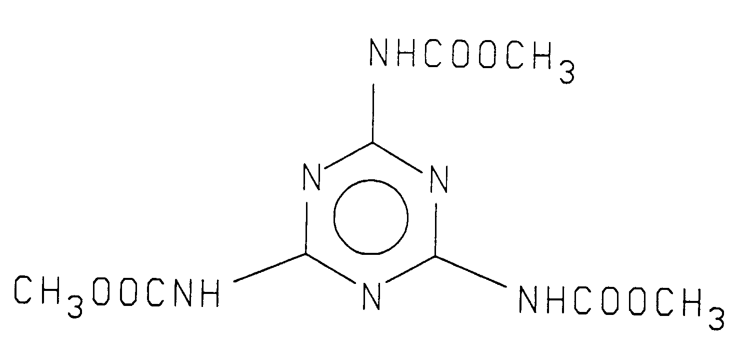
Another example resulting from the reaction of N,N',N''-trichloromelamine and 4-chlorobutyryl chloride is N,N',N''-tris(4-chlorobutyryl) melamine represented by the formula: 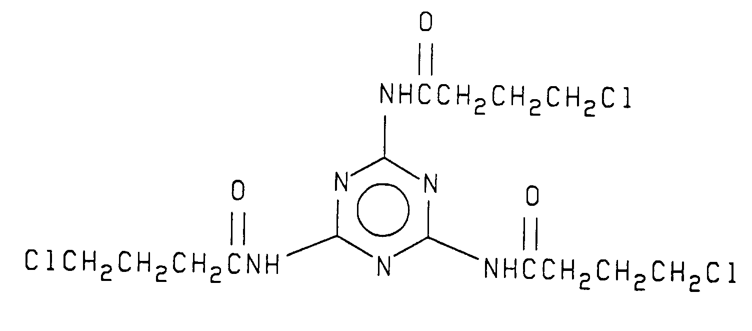
Another example of the acid amide products resulting from hexachloromelamine and methyl chloroformate is the triazine trichloro tris-carbamate represented by the formula: 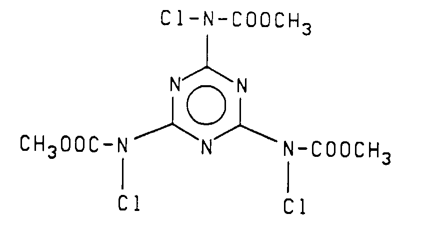
The trichloro tris-carbamate product may be readily reduced to the corresponding N-H compound using well known reducing agents such as methanol/triethylamine, hydrogen halide, or sulfur compounds. The reduction product is the triazine tris-carbamate and is identical with a sample previously prepared by the more direct reaction of N,N',N''-trichloromelamine and methyl chloroformate described above. The amine-methanol reduction step is depicted below:

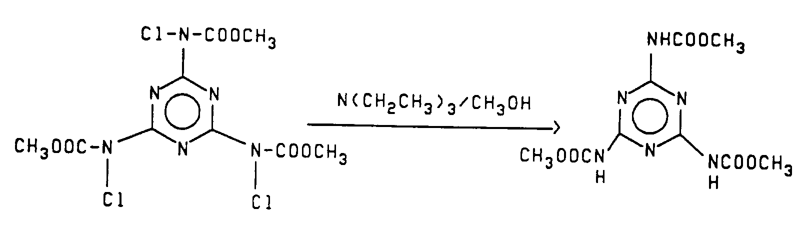

### THE NOVEL PROCESS

The novel process of the invention comprises contacting a haloamine with an acid halide at a temperature and for a length of time sufficient to produce an acid amide as a product and halogen as a by-product.

The preferred reaction temperature is in the range of from about-20^{o}C to about 120^{o}C, although higher or lower temperature may be employed. The reaction is typically carried out at about 60^{o}C, but any temperature at which no significant decomposition of starting materials occurs is generally suitable.

The preferred reaction time is in the range of from about 10 minutes to about 10 hours. The reaction is typically complete within a 2 to 5 hour period at about 60^{o}C.

The process may be carried out as a continuous or batch process. It is typically carried out by simply admixing, in any order, the haloamine and the acid halide reactants. Alternatively, an excess of one of the reactants may be used as a liquid medium to carry out the amide forming reaction. The liquid medium, however, is typically a solvent for the haloamine and the acid halide. The solvent is typically an aprotic solvent inert to chlorine or to the reactants. The reaction may also be carried out as a two phase reaction examplified as a liquid/solid two phase reaction.

The preferred solvent in the process of the invention is a halogenated solvent. Most preferably, the halogenated solvent is selected from the group consisting of methylene chloride, chloroform, carbon tetrachloride, 1,1,1- trichloroethane, chlorobenzene, ortho-dichlorobenzene, and a mixture of the least two of any of the aforementioned solvents.

The reactants may generally be mixed in varying amounts, however at least one equivalent of acid halide per one equivalent of haloamine is generally required. The use of excess acid halide is preferred, particularly when the acid halide used is volatile, and therefore easily removable by distillation, or when the acid halide is used as a solvent in which the amide forming reaction of the invention is carried out.

The reactants may be admixed with or without a solvent conveniently at room temperature, or at below ambient temperature particularly when the reaction is exothermic. In endothermic reactions, after admixing the reactants at room temperature, the reaction mixture is typically heated to accelerate product formation.

The reaction is usually carried out in an atmosphere of an inert gas under moisture free conditions. This minimizes the decomposition of reactants by atmospheric moisture and also minimizes the formation of undesirable hydrogen halide which may form from the reaction of moisture with the reactants.

Under the conditions of the process of the invention, the halogen by-product produced during the reaction is substantially free of hydrogen halide.

A rate accelerating catalyst such as trialkyl and triaryl phosphines, quaternary ammonium halides, or a monomeric or a polymeric 4-dialkylaminopyridine derivative may be added to the reactants, however, the process is typically carried out without adding a catalyst.

Suitable dialkylaminopypridine catalysts include 4-dimethylaminopyridine, 4-pyrrolidinopyridine, 4-piperidinopyridine, and poly-2-vinyl-4-dimethylaminopyridine (polydimethylaminopyridine). Suitable quaternary ammonium halides include ALIQUAT® 336 tricapryl methyl ammonium chloride, available from Kodak Laboratory Chemicals, Rochester, N. Y.

When the acid amide reaction product is obtained as a solution, a process further comprising isolating the reaction product by removing the volatiles under reduced pressure may be used. The volatiles may be removed by distillation. Alternatively, or after removing the volatiles and redissolving the residue in a solvent, the amide may be precipitated by adding a solvent in which the amide is substantially insoluble.

The product may be further purified by recrystallization, distillation, or chromatography.

### PROCESS FOR PRODUCING ACID AMIDES FROM MELAMINE BY RECYCLING THE HALOGEN BY-PRODUCT

It is the discovery of this invention that melamine may be converted to an acid amide by two different processes, each involving recycling of the halogen by-product. No input of additional halogen is required except to makeup for incidental halogen losses.

The conversion in the first novel process is via the trihalomelamine, and in the second novel process, the conversion is via the hexahalomelamine.

Both first and second processes are characterized by the following: the halogen by-product is recycled for use to convert additional melamine to hexahalomelamine. The two processes differ in that the first process comprises a step wherein a trihalomelamine is contacted with an acid halide, whereas the second process comprises a step wherein a hexahalomelamine is contacted with an acid halide.

The novel first and second processes comprising halogen recycle are described further below:

### FROM TRIHALOMELAMINE

The first method is a process for producing an acid amide, more specifically, a triazine tris-acid amide from melamine via the trihalomelamine comprising the steps of:
(a) contacting said melamine with a halogen to produce hexahalomelamine;
(b) contacting melamine with hexahalomelamine to produce trihalomelamine; and
(c) contacting said trihalomelamine with an acid halide at a temperature and for a length of time sufficient to produce triazine tris-acid amide as a product and halogen as a by-product.

Steps (a) and (b) of this process is carried out by the procedures disclosed in U.S. Pat. Nos. 2,472,361; 2,184,888; 2.184,886; and 2,184,883; and in Eur. Pat. No. 239,121.

Step (c) is carried out under conditions described previously hereinabove in the section entitled "THE NOVEL PROCESS".

Recycling is carried out by removing the halogen by-product by means such as a stream of a carrier inert gas and collecting the halogen vapors by bubbling through an aqueous caustic solution to form a hypohalite solution to be used as the halogenating agent. The preferred halogens which are best suited for recycling are chlorine and bromine. Chlorine is the most preferred halogen.

Alternatively, the halogen may be collected in an organic solvent and used as such to halogenate additional melamine.

### FROM HEXAHALOMELAMINE

The second method is a process for producing an acid amide, more specifically, a triazine tris- acid amide from melamine via the hexahalomelamine comprising the steps of:
(I) contacting said melamine with a halogen to produce hexahalomelamine;
(II) contacting said hexahalomelamine with an acid halide at a temperature and for a length of time sufficient to produce triazine N,N',N''- trihalo tris- acid amide as an intermediate product and halogen as a by-product; and
(III)contacting the triazine N,N',N''-trihalo tris- acid amide intermediate of step (II) with hydrogen halide to produce triazine tris- acid amide as a final product and additional halogen as a by-product.

Step (I) of this process is carried out by the procedures disclosed in U.S. Pat. Nos. 2,472,361; 2,184,888; 2,184,886; and 2,184,883.

Step (II) is carried out under conditions described previously hereinabove in the section entitled the "THE NOVEL PROCESS".

Step (III) is carried out by introducing hydrogen halide slowly, over a period of several minutes to several hours, into the reaction mixture at a temperature in the range of-20^{o}C to 120^{o}C.

The preferred hydrogen halides are hydrogen chloride and hydrogen bromide. Gaseous hydrogen chloride is the most preferred hydrogen halide.

Recycling is carried out by removing the halogen by-product from both steps (II) and (III) with a stream of a carrier inert gas and with collecting the halogen vapours by bubbling through an aqueous caustic solution to be used as the halogenating agent. The preferred halogens which are best suited for recycling are chlorine and bromine. Chlorine is the most preferred halogen.

Alternatively, the halogen may be collected in an organic solvent and used as such to halogenate additional melamine.

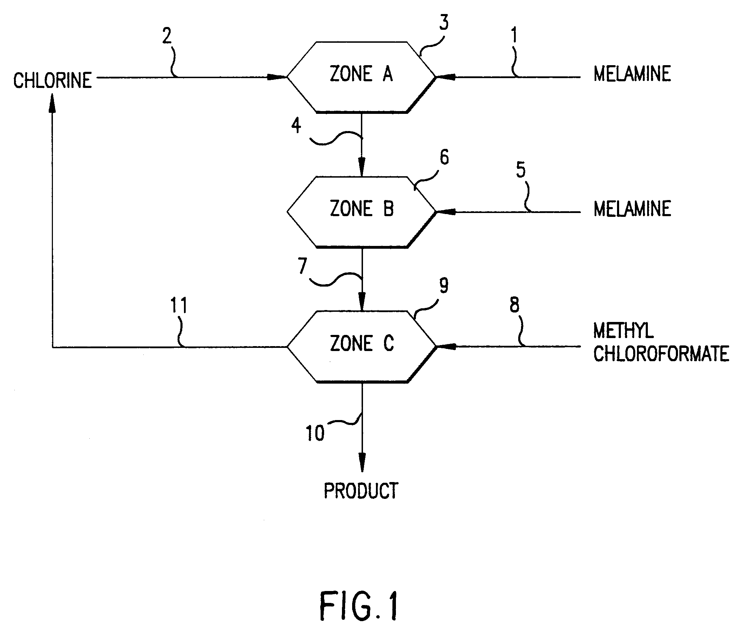

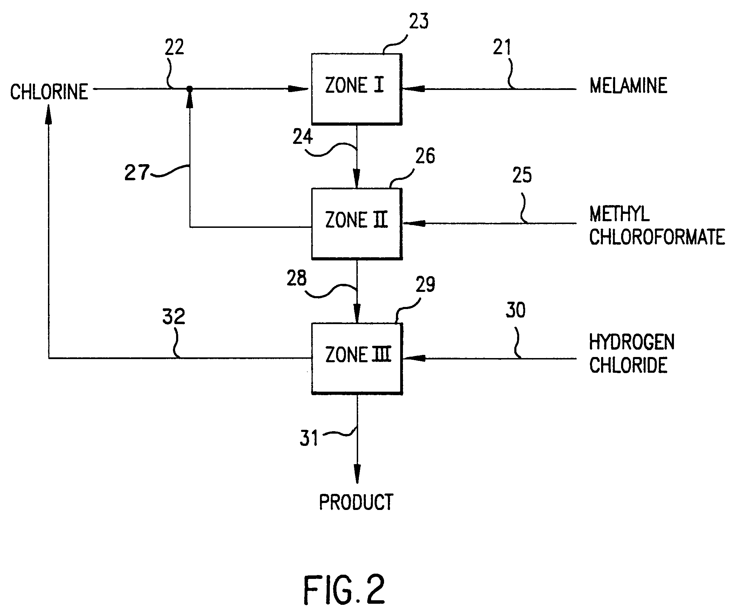

The process of the invention may be better understood by reference to FIGURE 1 and FIGURE 2 as follows:

### TRIS-CARBAMATE FROM TRISCHLOROMELAMINE (FIGURE 1):

Thus, in FIGURE 1 illustrating the process for producing triazine tris-methyl carbamate from melamine via trichloromalamine, melamine is introduced via line (1) and chlorine via line (2) into a reaction zone A (3) of the process and form hexachloromelamine.

Hexachloromelamine is then sent via line (4) and additional melamine via line (5) into reaction zone B (6) of the process to form trichloromelamine by a chlorine exchange reaction between melamine and hexachloromelamine.

Trichloromelamine is then sent via line (7) and methyl chloroformate via line (8) into a reaction zone C (9) of the process to form:
1. triazine tris-methyl carbamate as the product which is removed via line (10), and
2. chlorine gas as the by-product which is recycled via line (11) into Zone A (3).

### TRIS-CARBAMATE FROM HEXACHLOROMELAMINE (FIGURE 2):

In FIGURE 2 illustrating the process for producing triazine tris-methyl carbamate from melamine via hexachloromelamine, melamine is introduced via line (21) and chlorine via line (22) into reaction zone I (23) of the process to form hexachloromelamine.

Hexachloromelamine is then sent via line (24) and methyl chloroformate via line (25) into reaction zone II (26) of the process to form:
1. N,N',N''-trichloro triazine tris-methyl carbamate as an intermediate product to be reacted further, and
2. chlorine gas as the by-product which is recycled via line (27), to line (22), into zone I (23).

The intermediate product N,N',N''-trichloro triazine tris-methyl carbamate is then sent via line (28) and hydrogen chloride gas via line (30) into reaction zone III (29) of the process to form:
1. triazine tris-methyl carbamate as the final product which is removed via line (31), and
2. additional chlorine gas as the by-product which is recycled via line (32), to line (22), into zone I (23).

### UTILITY

Products prepared by the process of the invention have utility in diverse areas. In general, the utility of a particular product depends on its class.

### A. TRIAZINE TRIS-CARBAMATES

In curable compositions, members of the triazine tris-carbamate class have utility as cross-linking agents for polyfunctional active hydrogen compounds, including hydroxyfunctional and aminofunctional materials, as disclosed in detail in U.S. Pat. No. 4,939,213 cited previously hereinabove. The curable compositions disclosed therein may be used in solvent-based, water-based, or powder coatings or they may be used as aqueous dispersions which are particularly suited to application by electrodeposition. They are thus useful in catalyzed or uncatalyzed, one component heat cured systems, for applications such as coatings, particulary powder coatings, coil coatings, and can coatings. They are also usable in non-coatings applications such as conventional moldings, reactive injection moldings, composites, adhesives, and binders.

The triazine tris-carbamates of the invention have utility as intermediates leading to other crosslinkers. For example, they may be pyrolized to give triazine triisocyanates which themselves are excellent crosslinking agents as disclosed in U.S. Pat. No. 4,939,213.

The triazine tris-carbamates of the invention have utility as intermediates leading to polymer additives. They may be further converted to ultraviolet (UV) light stabilizers by substitution of at least one alkoxy or aryloxy group in the triazine tris-carbamates prepared by the process of this invention. The product may be a hindered amine light stabilizer (HALS).

For example, reaction of triazine tris-phenylcarbamate with 4-amino-2,2,6,6-tetramethylpiperidine at 25^{o}C to 160^{o}C temperature range produces, in good yield, a tris-urea derivative of melamine having the chemical name of tris-(2,2,6,6-tetramethylpiperid-4-yl-aminocarbonyl)melamine represented by the formula:

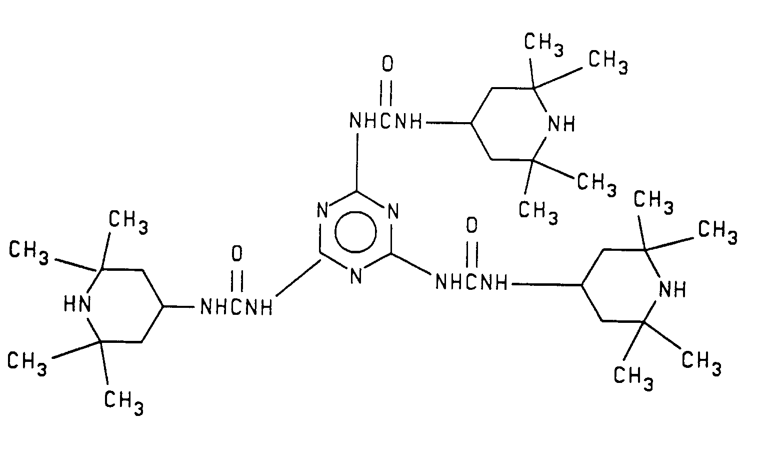

### B. TRIACYL MELAMINES

An example of triacyl melamine class is the N,N',N''-tristearylmelamine which has utility in polishing waxes and in water repellent fabric finishes as disclosed in U.S. Pat. No. 2,507,700.

### C. TRIS-(OMEGAHALOACYL) MELAMINES

Another class of compounds which may be prepared by the process of the invention is the tris-(omegahaloacyl)- and related melamine classes represented by the formula: 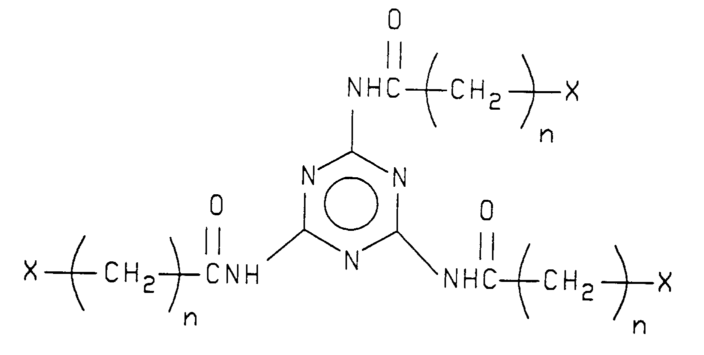
wherein
- n: is an integer from 1 to 5; and
- X: is a leaving group selected from the group consisting of chloride, bromide, iodide, fluoride, alkysulfonate, arylsulfonate, and mixtures thereof.

The preferred compounds are those wherein X is chloride and n is 3 or 4.

### D. LACTAM SUBSTITUTED TRIAZINE CROSSLIKERS

The tris-(omegahaloacyl) melamines of the invention are intermediates leading to the preparation of lactam-substituted triazine crosslinkers represented by the formula: 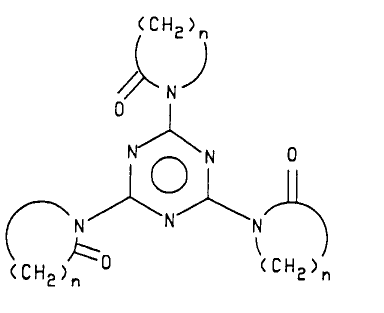
wherein
- n: is an integer from 1 to 5.

The preferred lactam-substituted triazine crosslinkers of the invention are those wherein n is 3 or 4.

### PREPARATION OF THE LACTAM CROSSLINKERS

The tris-lactam substitute triazine crosslinkers of the invention described in the immediately preceeding paragraphs above are easily prepared from the corresponding tris-(omegahaloacyl)-melamines via an intramolecular cyclization reaction upon the action of a strong base, typically in a non-protic solvent having a high dielectric constant for dissolving the various ionic intermediates at room temperature.

The intramolecular cyclization may be carried at about 0^{o}C to about 120^{o}C in a period of time in the range of from 10 minutes to 24 hours.

However, it is usually sufficient to carry out the intramolecular cyclization reaction at room temperature for a period of one to 6 hours only.

The product is isolated by adding a water-ice mixture to the reaction mixture to precipitate the lactam crosslinkers of the invention. Alternatively, the product may be extracted.

An example of tris-(omegahaloacyl)-melamine class is N,N',N''-tris-(4-chlorobutyryl)-melamine represented by the formula: 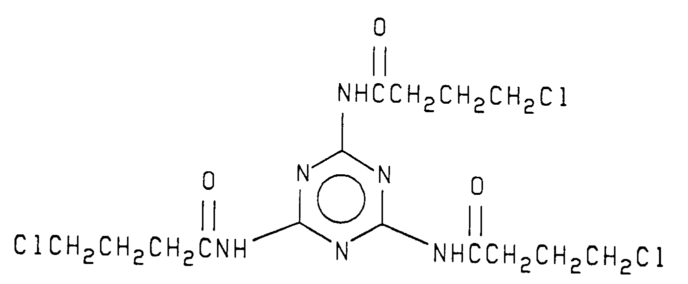
which has utility of being an intermediate leading to the N-substituted pyrrolidine crosslinking agent 2,4,6-tris(pyrrolidin-2-on-1-yl)-1,3,5-triazine, the utility of which as thermosetting resin material and hardening agents are disclosed in JP 58 146582. The tris-(N-pyrrolidonyl)-triazine is represented by the formula: 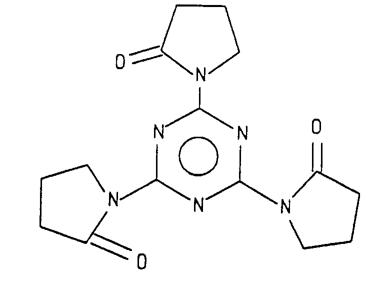
and may be easily prepared by a based-catalyzed intramolecular cyclization reaction of each of the 4-chlorobutyrylamino groups of the N,N'N''-tris-(4-chlorobutyryl) melamine precrusor.

### E. N-HALO ACID AMIDES

Another class of compounds which may be prepared by the process of the invention is N-halo acid amide intermediate products of the invention potentially useful as herbicides and pesticides as suggested by U.S. Pat. Nos. 3,920,832; 4,732,899; and 4,824,845.

A typical N-halo acid amide product of the invention is a composition of matter represented by formula: 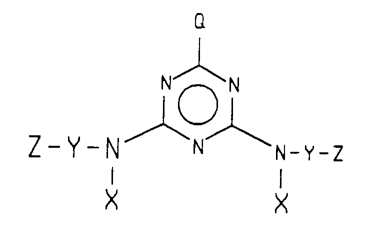
wherein
- Q: is 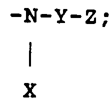 or
- Q: is selected from the group consisting of alkyl of 1 to 20 carbon atoms, alkenyl of 2 to 20 carbon atoms, aryl of 6 to 20 carbon atoms, aralkyl of 7 to 20 carbon atoms, alkoxy of 1 to 20 carbon atoms, aryloxy of 6 to 20 carbon atoms, alkylthio of 1 to 20 carbon atoms, arylthio of 6 to 20 carbon atoms, alkylamino of 1 to 20 carbon atoms, dialkylamino of 2 to 40 carbon atoms, morpholino, piperidino, pyrrolidino, hydrogen, chloro, bromo, iodo, fluoro, perfluoralkyl, perfluoroaryl, perfluoroaralkyl groups; and
wherein
- X: in each 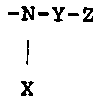 group is hydrogen or a halogen with the proviso that at least one X is a halogen independently selected from the group consisting of chloride, bromide, iodide, and fluoride; and
- Y: in each 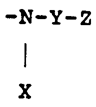 group is the same or different and each is independently selected from the group consisting of the following functionalities represented by the formula: 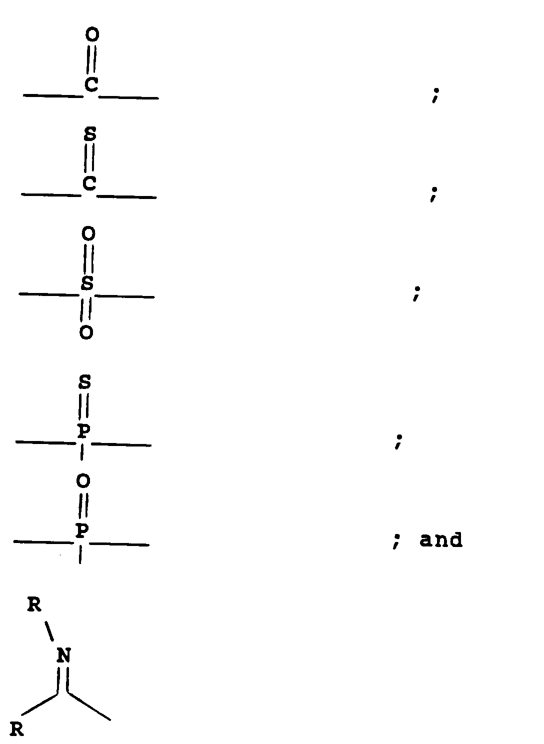 wherein each R is independently an alkyl, aryl, or an alkoxy group; and
- Z: is an M-functional anchor selected from the group consisting of monomeric or polymeric organic radical, hydrogen, chloro, bromo, iodo, fluoro, vinyl, alkyl of 1 to 20 carbon atoms, haloalkyl of 1 to 20 carbon atoms, aryl of 6 to 20 carbon atoms, haloaryl of 6 to 20 carbon atoms, aralkyl of 7 to 20 carbon atoms, haloaralkyl of 7 to 20 carbon atoms, acyl of 2 to 20 carbon atoms, haloacyl of 2 to 20 carbon atoms, halocarbonyl, alkoxycarbonyl of 2 to 20 carbon atoms, alkylthiocarbonyl of 2 to 20 carbon atoms, haloalkoxycarbonyl of 2 to 20 carbon atoms, aminocarbonyl, alkylaminocarbonyl of 2 to 20 carbon atoms, dialkylaminocarbonyl of 3 to 20 carbon atoms, alkenylaminocarbonyl of 4 to 20 carbon atoms, dialkenylaminocarbonyl of 7 to 40 carbon atoms, alkylalkenylaminocarbonyl of 5 to 20 carbon atoms, heteroaromatic ring, perfluoroalkyl of 1 to 20 carbon atoms, perfluoroaryl of 6 to 20 carbon atoms, perfluoro aralkyl of 7 to 20 carbon atoms, haloalkyl of 1 to 20 carbon atoms, alkoxycarbonylalkyl of 3 to 20 carbon atoms, cyanoalkyl of 2 to 20 carbon atoms, formylalkyl of 2 to 20 carbon atoms, ketoalkyl of 3 to 20 carbon atoms, trialkoxysilylalkyl of 4 to 20 carbon atoms, dialkylaminoalkyl of 3 to 20 carbon atoms, alkoxyalkyl of 2 to 20 carbon atoms, alkoxy of 1 to 20 carbon atoms, aryloxy of 6 to 20 carbon atoms, alkenyloxy of 3 to 20 carbon atoms, N,N-dialkylamino of 2 to 40 carbon atoms, N,N-dialkenylamino of 6 to 40 carbon atoms, N,N-diarylamino of 12 to 40 carbon atoms, N,N-alkylalkenylamino of 4 to 20 carbon atoms, N,N-alkylarylamino of 7 to 20 carbon atoms, N,N-alkenylarylamino of 9 to 20 carbon atoms, aziridino, azetidino, pyrrolidino, piperidino, morpholino, alkylmercapto of 1 to 20 carbon atoms, alkenylmercapto of 3 to 20 carbon atoms, and arylmercapto of 6 to 20 carbon atoms.

The products prepared by the process of the invention are obtained by contacting haloamine and acid chloride reactants under the process conditions described above. They may comprise carboxylic acid amides, carbamates, sulfonamides, phosphoramides, ureas, thioureas, thiophosphoramides, amidines, amidate esters, and mixtures thereof.

The following examples illustrates the various embodiments of the invention.

### EXAMPLE 1

### Triazine Trismethylcarbamate From Hexachloromelamine

A mixture of 3.33 g of hexachloromelamine, 23.6 g methyl chloroformate and 200 mg poly-dimethylaminopyridine was heated at 70^{o}C for 6 hours under argon. The excess methyl chloroformate was removed under reduced pressure. The residue was cooled and dissolved in a 50 ml methanol and 25 ml CH₂Cl₂ mixture. It was then treated with 5 ml triethylamine dropwise while cooling. The mixture was concentrated and the residue treated with methanol. Triazine trismethylcarbamate crystallized out which was filtered and characterized by ¹H NMR, ¹³C NMR, IR and Fast Atom Bombardment (FAB) mass spectroscopy (2.4 g; 80%);
m.p. greater than 300^{o}C; decomposition started at about 220^{o}C and peaked at about 250^{o}C;
¹H NMR (CDCl₃, delta): 3.8 (s, 9H, 3X OCH₃), 8.8 (s, 3H, 3X NH);
¹³C NMR (DMSO-d_{6,} delta): 52.3, 151.9, 164.9
IR (CHCl₃): 1760 cm⁻¹ (C = 0);
MASS (FAB, M+H⁺): 301.

### EXAMPLE 2

### Triazine Trismethylcarbamate From Trichloromelamine (Solventless Process)

A mixture of 2.3 g trichloromelamine, 20 ml methylchloroformate and 200 mg poly-dimethylaminopyridine was heated under argon at 75^{o}C for 3 hours. The excess methyl chloroformate was removed under reduced pressure. The residue was dissolved in a mixture of 100 ml methanol and 50 ml CH₂Cl₂. To it was added 1 ml Et₃N dropwise. It was filtered and solvent removed from the filtrate under reduced pressure. The residue on treatment with 20 ml methanol gave a crystalline product characterized to be triazine trismethylcarbamate (2.27 g; 76%), identical with the product by Example 1.

### EXAMPLE 3

### Triazine Trisbutylcarbamate From Trichloromelamine (Dichlorobenzene Solvent)

A mixture of 11.5 g trichloromelamine, 57.2 ml n-butylchloroformate and 50 ml o-dichlorobenzene was heated under Argon with stirring at 58-62^{o}C for 5 hours. It was allowed to cool to room temp. and diluted with 30 ml o-dichlorobenzene. 40 ml liquid was then collected by heating the reaction mixture at 55^{o}C under reduced pressure. The residue was cooled and diluted with 300 ml hexane. The precipitated material was then filtered and washed with 200 ml hexane. The residue was purified by silica gel column chromatography using CH₂Cl₂/MeOH (97:3) as the eluant. Triazine trisbutylcarbamate so obtained was identified by ¹H NMR, ¹³C NMR and mass spectroscopy (17.2 g; 81% yield);
m.p. 149-51^{o}C;
¹H NMR (CDCl₃, delta): 0.9(t, 9H, 3X CH₃CH₂CH₂CH₂O-),
1.3(m, 6H, 3X CH₃CH₂CH₂CH₂O-), 1.6 (m, 6H, 3 X CH₃CH₂CH₂CH₂O-),
4.1 (t, 6H, 3X CH₃CH₂CH₂CH₂O-), 8.7 (s, 3H, 3XNH);
¹³C NMR (CDCl₃, delta): 8, 20, 66, 150, 164;
MASS (FAB, M+H⁺): 427.

### EXAMPLE 4

### Triazine Trisphenylcarbamate From Trichloromelamine (Carbon Tetrachloride Solvent)

A mixture of 2.3 g. trichloromelamine, 10.0 ml phenyl chloroformate and 40 ml CCl₄ was heated under argon at 50^{o}C for 3 hours. It was cooled to room temperature and filtered. The residue was dissolved in a mixture of CH₂Cl₂/MeOH and solvent evaporated under reduced pressure. The residue was treated with anhydrous methanol and the precipitate formed was filtered and dried (4.2 g; 86% yield). The product thus obtained was characterized to be triazine trisphenylcarbamate by ¹H NMR, ¹³NMR, IR and mass spectroscopy;
Deblocking (decomposition) started at about 100^{o}C and peaked at about 160^{o}C;
¹H NMR (DMSO-d₆, delta): 7.2 - 7.5 (m, 15H, 3X ArH₅), 11.2 (s, 3H, 3XNH);
¹³C NMR (DMSO- d₆, delta): 122, 126, 129, 149, 151, 165;
MASS (FAB, M+H⁺): 487.

### EXAMPLE 5

### N, N',N''-Tris(4-Chlorobutyryl) Melamine

A mixture of 2.3 g trichloromelamine, 20 ml carbon tetrachloride, 8.46 g 4-chlorobutyryl chloride and 30 mg N,N-dimethylaminopyridine was placed in a 100 ml flask fitted with a magnetic stirring bar, a reflux condenser and argon inlet. The reaction mixture was slowly heated in an oil bath to 60^{o}C and was stirred at 60^{o}C for 5 hours. It was then allowed to cool down to room temperature and diluted with 50 ml hexanes. The contents were stirred at room temperature for 30 minutes and then filtered. The residue was washed with hexane and dried under reduced pressure. It was characterized to be N,N',N''-tris(4-chlorobutyryl) melamine on the basis of NMR and mass spectroscopy (4.2 g; 95% yield);
¹H NMR (DMSO-d₆, delta): 2.0 (m, 6H, 3X CH₂CH₂CH₂Cl),
2.8 (t, 6H, 3X NHCOCH₂CH₂CH₂Cl), 3.6 (t, 6H, 3X CH₂CH₂Cl), 11.8 (broad s, 3H, 3X NHCO);
¹³C NMR (DMSO-d₆, delta): 27, 34, 44, 161, 174;
MASS (FAB, M+H⁺): 439.

### EXAMPLE 6

### N,N',N'',-Trischloroacetyl Melamine

460 mg trichloromelamine was placed in a 3-neck 100 ml flask fitted with a reflux condenser, argon inlet, a magnetic stirring bar, and a rubber septum inlet. To the flask was added 10 ml carbon tetrachloride followed by 1.4 ml chloroacetylchloride with a syringe under stirring. The reaction mixture was heated up to 60^{o}C in an oil bath for 4.5 hours. It was then allowed to cool to room temperature and the excess reagent and carbon tetrachloride were removed under reduced pressure. The residue was diluted with hexane and the precipitated material filtered off, washed with hexane and dried under reduced pressure to give a product (689 mg; 97% yield) which was characterized to be N,N',N''-trischloroacetyl melamine based on ¹H NMR, ¹³C NMR and mass spectroscopic data;
¹H NMR (DMSO-d₆, delta): 4.8 (s, 6H, 3X CH₂Cl), 11.1 (s, 3H, 3X NHCO);
¹³C NMR (DMSO-d₆, delta): 45.8, 163.8, 167.1;
MASS (FAB, M+H⁺): 355.

### EXAMPLE 7

### Triazine Trisethyl Carbamate

The procedure of Example 2 was repeated at 80^{o}C using ethyl chloroformate (20.0 ml) in the place of methyl chloroformate. The product was triazine tris-ethyl carbamate( 2.62g, 76% yield). m.p. greater than 300^{o}C; decomposition started at about 200^{o}C and peaked at about 260^{o}C.

### EXAMPLE 8

### Triazine Trispropyl Carbamate

The procedure of Example 4 was essentially repeated using propyl chloroformate (10.0 ml) in the place of phenyl chloroformate. The product was triazine tris-propyl carbamate. 2.3 g of trichloromelamine was placed in a 250 ml flask equipped with a magnetic stirring bar, a reflux condenser and an argon inlet. To it was added 50 ml CCl₄ followed by 10.0 ml propylchloroformate. The reaction mixture was gradually heated in an oil bath to 75^{o}C and kept at 75^{o}C for 6 hours. Removal of the excess propyl chloroformate followed by usual workup gave a product which was characterized to be triazine tris-propyl carbamate by ¹H NMR, ¹³C NMR, IR and mass spectroscopy; m.p. 178 to 182^{o}C.

### EXAMPLE 9

### Triazine Tris-(2-chloroethylcarbamate)

The procedure of Example 4 was repeated using 2-chloroethyl chloroformate (3.5ml) in the place of phenyl chloroformate. The product was triazine tris-(2-chloroethylcarbamate). The procedure was as follows:
To a stirring suspension of 1.15 g trichloromelamine in 30 ml CCl₄, in a 100 ml flask fitted with a reflux condenser, a magnetic stirring bar and argon inlet was added 3.5 ml (4.8 g) of 2-chloroethyl chloroformate. The reaction mixture was stirred at room temperature for 0.5 hours and then slowly heated in an oil bath to 75^{o}C. The heating was continued for 6 hours and the reaction was then allowed to cool to room temperature. Excess 2-chloroethyl chloroformate and CCl4 were removed under reduced pressure and the residue purified by column chromatography (silica gel) using CH₂Cl₂ and MeOH mixture (95:5) as the eluant to give triazine tris(2-chloroethyl) carbamate as characterized by ¹H NMR, ¹³C NMR, mass and IR spectroscopy; m.p. n 130^{o}C (decomp.)

### EXAMPLE 10

### Triazine Tris-(2-ethylhexylcarbamate)

The procedure of Example 4 was repeated using 2-ethylhexyl chloroformate (86.7g) in the place of phenyl chloroformate. The product was triazine tris-(2-ethylhexylcarbamate). The procedure was as follows: A mixture of 11.5 g trichloromelamine, 150 ml o-dichlorobenzene and 86.7 g 2-ethylhexyl chloroformate was heated in a flask equipped with a magnetic stirring bar, an Argon inlet and a reflux condenser. The temperature of the oil bath was gradually increased to 67^{o}C. After 6 hours at 67^{o}C, the heating was discontinued and the reaction mixture concentrated under reduced pressure. Usual work-up of the reaction mixture gave a product which was characterized to be triazine tris-(2-ethylhexylcarbamate) on the basis of spectroscopic data.

### EXAMPLE 11

### Triazine Tris-(iso-butylcarbamate)

The procedure of Example 3 was slightly modified and isobutyl chloroformate (16.4 ml) was used in the place of n-butyl chloroformate. The product was triazine tris-(iso-butylcarbamate). The slightly modified procedure was as follows:
To a flask equipped with a magnetic stirring bar, a reflux condenser and an Argon inlet, 4.6 gm of trichloromelamine was added followed by 40 ml o-dichlorobenzene and 16.4 gm isobutylchloroformate. The reaction mixture was heated in an oil bath at 65 - 70^{o}C for 6 hours. It was then allowed to cool and excess isobutyl chloroformate and o-di- chlorobenzene were removed under reduced pressure. The residue was treated with 100 ml hexanes and the precipitated material filtered and washed with 50 ml hexane and dried. The product was characterized to be triazine tris-isobutyl carbamate by ¹H NMR, ¹³C NMR, IR, and mass spectroscopy. It decomposed starting at about 230^{o}C and peaked at about 245^{o}C.

### EXAMPLE 12

### Triazine Mixed Alkyl Carbamates

The procedure of Example 3 was essentially repeated using a mixture of haloformates consisting of 2-ethylhexyl chloroformate (4 ml) and methyl chloroformate (6.2 ml) in the place of n-butyl chlorformate. The product was a triazine tris-(mixed alkyl carbamate) wherein the mixed alkyl group consisted of 2-ethylhexyl and methyl groups in ratio in the range of from about 1:1.8 to about 1:0.77. The detailed experimental procedure was as follows:
A mixture of 4.6 g trichloromelamine, 4 ml 2-ethylhexyl chloroformate and 30 ml o-dichlorobenzene was heated under Argon with stirring at 60^{o}C for 4 hrs. It was allowed to cool at room temperature. Then 6.2 ml of methyl chloroformate was added. The reaction mixture was then heated under Argon with stirring to 65^{o}C for 2 hours. It was allowed to cool to room temperature and diluted with 15 ml of dichlorobenzene; 10.5 ml liquid was then collected by heating the reaction mixture to 40^{o}C under reduced pressure. The residue was cooled and diluted with 200 ml hexanes. The precipitated material was filtered and washed with 300 ml hexanes.

Five gram of the solid was stirred with 250 ml methylene chloride and 2.6 g of insolubles were filtered off and identified to be the N-(2-ethylhexoxycarbonylamino)-N', N''-di(methoxycarbonylamino) triazine with an ethylhexyl/methyl ratio of 1:1.8 by ¹H NMR (52% yield). The methylene chloride solution was washed 2 times with 5% sodium bisulfite and 2 times with deionized water, dried over magnesium sulfate and stripped of solvent to give 1.86 gram of N-(ethylhexoxycarbonylamino)-N'-(methoxycarbonylamino) triazine with a ethylhexyl/methyl ratio of 1.3:1 (or 1:0.77) as identified by ¹H NHR (37% yield).

### EXAMPLE 13

### N,N',N''-Tris-(3-propionyl) Melamine

To a stirring suspension of 2.3 g trichloromelamine in 40 ml CCl₄ was added 7.62 g 3-chloropropionyl chloride under Argon. The reaction mixture was heated in an oil bath at 65-70^{o}C for 6 hours. The reaction mixture was cooled and then diluted with 50 ml hexane. The precipitates were filtered, washed with hexane and dried under reduced pressure (3.9 g; 98% yield). The product thus obtained was characterized to be N,N',N''-tris(3-chloropropionyl) melamine by ¹H NMR, ¹³C NMR, IR, and mass spectroscopy.

### EXAMPLE 14

### N,N',N''-Triacetyl Melamine

The procedure of Example 6 was repeated at room temperature for 20 hrs, using acetyl chloride (15 ml) in the place of chloroacetyl chloride. The product was triacetyl melamine.

### EXAMPLE 15

### N,N',N''-Trihexanoyl Melamine

The procedure of Example 6 was essentially repeated using hexanoyl chloride (8.07g) in the place of chloroacetyl chloride. The product was trihexanoyl melamine. The experimental procedure was as follows:
A mixture of 2.3 g trichloromelamine, 8.07 g hexanoyl chloride, and 25 ml carbon tetrachloride was heated with stirring under Argon for 6 hours at 70-78^{o}C. The reaction mixture was allowed to cool to room temperature and the product was precipitated by adding 100 ml hexane. The precipitated material was filtered, washed with 50 ml hexane and dried. Spectroscopic data were consistent with N,N',N''-trihexanoyl melamine structure.

### EXAMPLE 16

### N,N',N''-Tributyryl Melamine

The procedure of Example 6 was essentially repeated using butyryl chloride (6.4 g) in the place of chloroacetyl chloride. The product was tri-butyryl melamine. The procedure was as follows:
2.3 g of trichloromelamine was treated with 6.4 g butyryl chloride in 40 ml CCl₄ at 65^{o}C for nearly 6 hours. The reaction mixture was cooled and diluted with 100 ml hexane. The precipitated material was filtered, washed with hexane and dried. Mass spectroscopic analysis indicated that the product was N,N',N''-tributyryl melamine.

### EXAMPLE 17

### N,N',N''-Tribenzoyl Melamine

The procedure of Example 6 was essentially repeated using benzoyl chloride (6.7 ml) in the place of chloroacetyl chloride. The product was tribenzoyl melamine. The procedure was as follows:
To a stirring suspension of 2.3 g of trichloromelamine in 40 ml CCl₄ in a flask equipped with a magnetic stirring bar, an Argon inlet and a reflux condenser was added 6.7 ml benzoyl chloride. The reaction mixture was heated in an oil bath at 68-70^{o}C for 7 hours. The reaction mixture was then allowed to cool and then diluted with 30 ml hexane. The precipitated material was filtered off and the residue washed with 150 ml hexane and then dried under reduced pressure. (4.3 g; 98% yield). The product was characterized to be N,N',N''-tribenzoyl melamine by ¹H NMR, ¹³C NMR, IR and mass spectroscopy.

### EXAMPLE 18

### N,N',N''-Tri-paranitrobenzoyl Melamine

The procedure of Example 6 was essentially repeated using para-nitrobenzoyl chloride (5.55 g) in the place of chloroacetyl chloride. The product was tri-paranitrobenzoyl melamine. The procedure was as follows:
To a stirring suspension of trichloromelamine (1.15 g) in CCl₄ (20 ml) was added a solution of 5.55 g of p-nitrobenzoyl chloride in 30 ml CCl₄. The reaction mixture was heated in an oil bath under an atmosphere of Argon at 70^{o}C for 6 hours, was allowed to cool and then was diluted with 25 ml CCl₄. The precipitates were filtered, washed with 100 ml CCl₄ and dried under reduced pressure. The product was characterized to be N,N'N''- tri-paranitrobenzoyl melamine by ¹H and ¹³C NMR spectroscopy.

### EXAMPLE 19

### Tris-(2,2,6,6-tetramethylpiperidin-4-yl-aminocarbonyl)melamine

A mixture of the triazine tris-phenylcarbamate (4.86 g) product of Example 4 and 4-amino-2,2,6, 6-tetra-methylpiperidine (15.4ml) in toluene solvent (80 ml) was heated at 115^{o}C for 8 hours. After cooling, addition of hexane (100 ml), a tris-urea derivative characterized to be tris-(2,2,6,6-tetramethylpiperidin-4-yl-aminocarbonyl) melamine was obtained (5.95 g, 89% yield). It decomposed starting at about 220^{o}C and peaked at about 270^{o}C.

### EXAMPLE 20

### Preparation of 2,4,6-Tris(pyrrolidin-2-on-1-yl)-1,3, 5-triazine from N,N',N''-Tris(4-chlorobutyryl)melamine

Sodium hydride (200 mg, 60% in mineral oil) was placed in a 3- neck flask fitted with an Argon inlet, a stopper, a rubber septum and a magnetic stirring bar. To it was added 5 ml n-hexane and the mixture allowed to stir for several minutes. Stirring was stopped and with the help of a syringe, n-hexane was removed. To the washed NaH thus obtained was added 5 ml dimethylformamide (DMF). The flask was cooled to 0^{o}C in an ice-bath and 440 mg of N,N',N''-tris(4-chlorobutyryl)melamine, the product of Example 5, dissolved in 5 ml DMF was added with stirring to the flask containing NaH. The reaction mixture was stirred at 0^{o}C for 5 hours. The cooling bath was then removed and the reaction mixture allowed to warm to room temperature. The reaction mixture was then slowly added to 100 ml ice-cold water. The reaction mixture was extracted with CH₂Cl₂ (3 X 30 ml) and the combined organic extract washed with water (20 ml) and dried over MgS0₄ (anhydrous). The MgS0₄ was filtered and the filtrate concentrated under reduced pressure. The solvent was then removed and residue was dried under reduced pressure. The product was essentially a pure compound (270 mg, 82% yield) and was characterized to be 2,4,6-tris-(pyrrolidin-2-on-1-yl)-1,3,5-triazine by NMR and mass spectroscopy;
¹H NMR (CDCl₃,delta): 2.0 (m, 6H, 3XCH₂CH₂-CH₂CO), 2.6 (t, 6H, 3X CH₂CH₂CO), 4.0 (t, 6H, 3X NCH₂CH₂);
MASS (FAB, M+H⁺): 331.

### EXAMPLE 21

A mixture of 1.15g trichloromelamine, 30ml CCl₄, 4.2g benzoyl chloride and 50mg ALIQUAT® 336 (tricaprylyl- methylammonium chloride) in 0.5ml CCl₄ was stirred at room temperature. Formation of chlorine indicated by the formation of yellow color was noticed within 20 minutes. Thin layer chromatographic analysis of the reaction mixture after 30 minutes indicated the formation of N,N',N''-tri- benzoyl melamine when compared directly with the previously prepared N,N',N''-tribenzoyl melamine.

In a second experiment, 1.15g trichloromelamine was stirred in 30ml CCl₄ with 4.2g benzoyl chloride at room temperature, with no catalyst added. In this case no clor was observed in 20 minutes. Furthermore, thin layer chromatographic analysis after 30 minutes and even after 4 hours indicated the absence of products corresponding to N,N',N''-tribenzoyl melamine.

It is concluded from these comparative experiments that the reaction of the invention is catalyzed by chloride ions.

### EXAMPLE 22

The procedure of Example 4 was essentially repeated using hexabromomelamine (5.2g), prepared by the procedure described in U.S. Pat. No. 2,472,361.

The experimental details were as follows:

### PART A: Preparation of Hexabromomelamine

In accordance to the procedure outlined by W. C. Arsem in US 2,472,361:
4.1 grams of melamine was suspended in 100 grams of deionized water and 24.1 grams of glacial acetic acid was added. The mixture was cooled to about 5^{o}C and to it was added with stirring an aqueous solution containing 25 grams of sodium hypobromite in approximately 5 percent concentration over a period of 2 hours. Additional acetic acid was added during the course of the reaction to keep the pH below 7. Thirty minutes after completion of the NaOBr addition, the solid product was filtered off, washed with 700 ml of water, and dried under reduced pressure at room temperature. The product was a dark yellow powder (14.85 g; 88% yield) which was characterized by ¹³C NMR and IR. Titration for active bromine content gave a value of 8.97 mmol bromine per gram of product, which is 89.6% of the theoretical value.

### PART B: Preparation of N,N',N''-Tribromo-2,4,6-Tris-methoxycarbonylaminotriazine from Hexabromomelamine in Carbon Tetrachloride

A mixture of 5.2 grams of hexabromomelamine, 9.45 grams of methyl chloroformate, and 50 ml of carbon tetrachloride was heated with stirring at 55-75^{o}C for five hours under a nitrogen atmosphere. The evolution of BrCl vapor was observed during the course of the reaction. After cooling, the solid product was filtered off, washed with 200 ml of CCl₄, and dried under reduced pressure at room temperature.

The product was a yellow powder (2.30 g; 43% yield) which was analyzed by ¹H NMR, ¹³C NMR, and IR. Titration for active bromine content gave a value of 5.85 mmol bromine per gram of product, which corresponds to 104.6% of the theoretical value. From the spectroscopic analysis and from the results of the titration, it was concluded the the product was N,N',N''-tribromo-N,N',N''-tris(methoxycarbonylamino-1,3,5-triazine).

### EXAMPLE 23

### PART A: N,N,N'-Trichlorobenzoguanamine from Benzoguanamine

A mixture of 18.72g benzoguanamine, 24.25 ml glacial acetic acid, and 350 ml deionized water was cooled at 9^{o}C in an ice bath under Argon. To this was added with stirring 641 ml of 5% aqueous sodium hypochlorite over a period of 70 minutes at 9^{o}C. The mixture was stirred for half an hour, filtered, and the residue washed with 700 ml deionized water. The solids were dried at room temperature under reduced pressure to give N,N,N'-trichlorobenzoguanamine The product was identified by ¹H NMR and IR spectroscopy and by chlorine titration to be the N,N,N'-trichlorobenzoquanamine (93% of the theoretical value of active chlorine); (27.93 g, 91.7% yield).

### PART B: Preparation of 2,4-Di(butoxycarbonylamino)-6-phenyl-1,3,5-triazine from Trichloro Benzoguanamine.

A mixture of 2.5 grams of N,N,N'-trichlorobenzoguanamine, 5.5 ml butyl chloroformate, and 20 ml 0-dichlorobenzene was heated under Argon with stirring at 70^{o}C for 8 1/2 hours. It was cooled to room temperature and 125 ml of hexane was added. The mixture was filtered and the solids dried under reduced pressure at room temperature. The product was characterized to be 2,4-di(butoxycarbonylamino)-6-phenyl-1,3,5-triazine by ¹H NMR, ¹³C NMR, active chlorine titration and TLC; (2.5 g, 90.5%).

### EXAMPLE 24

### PART A: Preparation of N,N,N',N'-Tetrachloroacetoguanamine

Acetoguanamine as a fine powder (12.5 grams) was mixed with 200 grams of deionized water and 27 grams of glacial acetic acid. The mixture was then cooled to about 13^{o}C and to it was added with stirring an aqueous solution containing 33.5 grams of sodium hypochlorite in approximately 5 percent concentration over a period of one hour. Additional acetic acid was occasionally added during the course of the reaction to keep the solution pH below 7. Thirty minutes after completion of the NaOCl addition, the solid product was filtered off, washed with 700 ml of water, and dried under reduced pressure at room temperature to give a light yellow powder (22.65 g; 86% yield) characterized by ¹H NMR, ¹³C NMR, and IR to be N,N,N',N'-tetrachloroacetoguanamine. Titration for active chlorine content gave a value of 14.64 mmol chlorine per gram of product, which corresponds to 96.2% of the theoretical value.

### PART B: Preparation of N-Chlorinated 2,4-Di(butoxycarbonylamino)-6-methyl-1, 3,5-triazines from N,N,N',N'-Tetrachloroacetoguanamine and Butyl Chloroformate

A mixture of 2.5 grams N,N,N',N'-tetrachloroacetoguanamine, 6 ml of n-butyl chloroformate, 20 ml of o-dichlorobenzene, and 30 milligrams of N,N-dimethylaminopyridine was heated under Argon with stirring for 24 hours at 68^{o}C. The reaction solution was then cooled to room temperature and the excess reagent and o-dichlorobenzene were removed under reduced pressure.

The resulting yellow-orange solid (2.15 g) was found by ¹H and ¹³C NMR, and by TLC analysis to be a mixture of unreacted, mono-butoxycarbonylamino (22%), and di-butoxycarbonylamino (16%) chloro acetoguanamines.

### PART C: Preparation of 2,4-Di(butoxycarbonylamino)-6-methyl-1,3,5-triazine from N,N,N',N-Tetrachloroacetoguanamine and Butyl Chloroformate

A mixture of 2.5 g N,N,N',N'-tetrachloroacetoguanamine, 6 ml of n-butyl chloroformate, 20 ml of o-dichlorobenzene, and 0.12 g of Aliquat® 336 was heated under argon with stirring for 24 hours at 68^{o}C. Upon cooling, 100 ml of hexane was added. The resulting precipitate was isolated, dried under reduced pressure (1.65 g, 56.3% yield), and identified by ¹H NMR, ¹³C NMR, IR, TLC, and active chlorine analysis to be 2,4-di(butoxycarbonylamino)-6-methyl-1,3,5-triazine.

### EXAMPLE 25

### Preparation of N,N',N''-Triacetylmelamine from N,N',N''-Trichloromelamine and Acetyl Bromide

A mixture of 1.38 grams of N,N',N''-trichloromel- amine, 5.90 grams of acetyl bromide, and 40 ml of carbon tetrachloride was heated with stirring under Argon for 2 hours at 61^{o}C. An evolution of a reddish-brown vapor (BrC1) was observed during the course of the reaction. After cooling, the orange solid was filtered, washed several times with CC1₄ and dried under reduced pressure at room temperature to afford a white powder (0.76 g, 50% yield).

The recovered product was characterized by ¹H NMR, ¹³C NMR, and TLC analysis to be N,N'N''-triacetylmelamine, identical with the product of Example 14.

### EXAMPLE 26

### Debromination of N,N',N''-Tribromo-2,4,6-tri(methoxycarbonylamino)-1,3,5-Triazine with Hydrogen Chloride: Preparation of the 2,4,6-Tri(methoxycarbonylamino-1,3,5-Triazine Hydrochloride Salt

A slurry of 0.98 grams of the material from Example 22, Part B, in 50 ml of carbon tetrachloride was cooled to 17^{o}C with an external water bath. This mixture was then saturated with anhydrous hydrogen chloride and allowed to stir for one hour. The solution color deepened during the course of the reaction from yellow to reddish orange. After one hour, the solid product was filtered, washed several times with CCl₄, and air dried overnight.

The product was a lightly yellow tinted powder (0.66 grms, 98% yield) characterized to be the HC1 salt of 2,4,6-trimethoxycarbonylamino)-1,3,5-triazine by ¹H and ¹³C NMR.

### EXAMPLE 27

### PART A: Preparation of N,N'-Dichloroacetoguanamine

A slurry of N,N,N',N'-tetrachloracetoguanamine (5.3 g), acetoguanamine (2.5 g), deionized water (90 ml) and glacial acetic acid (0.2 ml) was heated with stirring at 50^{o}C under nitrogen for one hour. Upon cooling, the solid product was filtered off, washed with 140 ml of water, and dried under reduced pressure at room temperature to give a white power (7.4 g, 95% yield) characterized by ¹H NMR, ¹³C NMR and IR to be N,N'-dichloroacetoguanamine. Titration for active chlorine content given a value of 10.19 mmol chlorine per gram of product, which corresponds to 98.8% of the theoretical value. m.p. 186-187^{o} (dec).

### PART B: Preparation of 2,4-Di(butoxycarbonylamino)-6-methyl-1,3,5-triazine from N,N'-Dichloroacetoguanamine and Butyl Chloroformate

A mixture of 2.5 g N,N'-dichloroacetouganamine, 8.3 ml n-butyl chloroformate, and 20 ml of o-dichlorobenzene was heated with stirring at 70^{o}C under Argon for five hours. Upon cooling, the insoluble solids were filtered off and washed with hexanes. The combined hexane/o-dichlorobenzene filtrate was concentrated under reduced pressure. The resulting colorless residue (2.3 g, 55% yield) was characterized by IR, TLC, ¹H NMR, ¹³C NMR, and active C1 analysis to be 2,4-di(butoxycarbonylamino)-6-methyl-1,3,5-triazine, identical with the product of Example 24, Part C.

### EXAMPLE 28

### Reaction of Trichloromelamine with Benzoyl Chloride Using 4-Dimethylamino pyridine (DMAP) as Catalyst

To a stirring suspension of 1.15 g trichloromelamine in 30 ml CCl4 was added 4.2 g benzoyl chloride followed by 40 mg 4-dimethylaminopyridine The reaction mixture was stirred at room temperature under Argon. After 0.5 hours the reaction mixture turned yellowish. A thin layer chromatographic analysis revealed the formation of tribenzoyl melamine as one of the products by comparison with an authentic sample of tribenzoyl melamine.

In a control experiment without the catalyst but under the same reaction conditions as above, formation of the tribenzoyl melamine was not observed after 0.5 hours. It is concluded from the above comparative experiments that 4-dimethylaminopyridine is an effective catalyst for the novel reaction of the invention.

Although the present invention has been described with reference to certain preferred embodiments, it is apparent that modifications and variations thereof may be made by those skilled in the art without departing from the scope of this invention as defined by the appended claims.

## Claims

1. A process for preparing an acid amide from a haloamine and an acid halide comprising: contacting said haloamine with said acid halide at a temperature and for a length of time sufficient to produce said acid amide as a product and halogen as a by-product.

2. The process for preparing an acid amide from a haloamine and an acid halide as recited in claim 1 wherein the haloamine is represented by the formula: 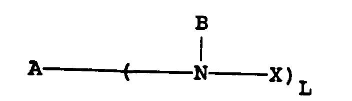 wherein
L is at least 1; and when L is at least 2, each 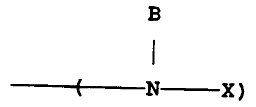 group is the same or different;
X is a halogen selected from the group consisting of chloro, bromo, iodo, fluoro groups, and mixtures thereof.
B is a group selected from the group consisting of hydrogen, chloro, bromo, iodo, fluoro, aminocarbonyl, alkylaminocarbonyl of 2 to 20 carbon atoms, dialkylaminocarbonyl of 3 to 40 carbon atoms, heteroaromatic ring, alkylthiocarbonyl, silyl, cyano, perfluoroalkyl, perfluoroaryl, and perfluoroaralkyl groups; and
A is an L functional anchor selected from the group consisting of monomeric organic radical, a polymeric organic radical, hydrogen, and halogen groups.

3. The process for preparing an acid amide from a haloamine and an acid halide as recited in claim 2 wherein the L-functional A is an anchor selected from the group consisting of hydrogen, chloro, bromo, iodo, fluoro, aminocarbonyl, alkylaminocarbonyl of 2 to 20 carbon atoms, dialkylaminocarbonyl of 3 to 40 carbon atoms, heteroaromatic ring, alkylthiocarbonyl, silyl, cyano, perfluoroalkyl, perfluoroaryl, perfluoroaralkyl phthaloyl, succinoyl, maleoyl, ortho-phenylenedisulfonyl, 1,2-ethylenedisulfonyl, anchors represented by the following formulae: 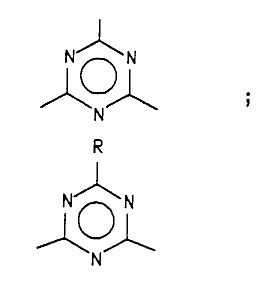 wherein
R is selected from the group consisting of alkyl of 1 to 20 carbon atoms, alkenyl of 2 to 20 carbon atoms, aryl of 6 to 20 carbon atoms, aralkyl of 7 to 20 carbon atoms, alkoxy of 1 to 20 carbon atoms, aryloxy of 6 to 20 carbon atoms, alkylthio of 1 to 20 carbon atoms, arylthio of 6 to 20 carbon atoms, alkylamino of 1 to 20 carbon atoms, dialkylamino of 2 to 40 carbon atoms, morpholino, piperidino, pyrrolidino, hydrogen, chloro, bromo, iodo, fluoro, perfluoroalkyl, perfluoroaryl, and perfluoroaralkyl group; 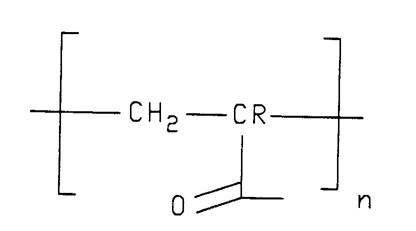
wherein
R is selected from the group consisting of hydrogen, alkyl, and aryl groups; and
n is at least 2; 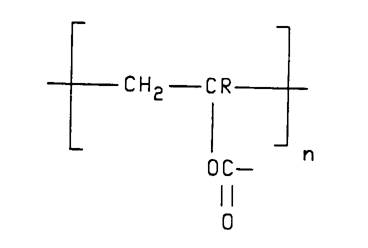
wherein
R is selected from the group consisting of hydrogen, alkyl and aryl groups; and
n is at least 2;
and mixtures thereof.

4. The process for preparing an acid amide from a haloamine and an acid halide as recited in claim 3 wherein A is a polyfunctional anchor group selected from the group consisting of the following structures represented by the formulae: 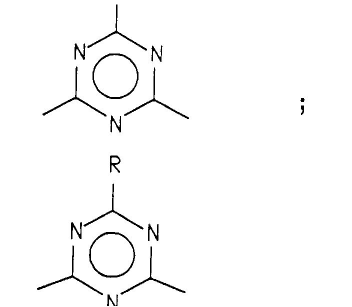 wherein
R is selected from the group consisting of alkyl of 1 to 20 carbon atoms, alkenyl of 2 to 20 carbon atoms, aryl of 6 to 20 carbon atoms, aralkyl of 7 to 20 carbon atoms, alkoxy of 1 to 20 carbon atoms, aryloxy of 6 to 20 carbon atoms, alkylthio of 1 to 20 carbon atoms, arylthio of 6 to 20 carbon atoms, alkylamino of 1 to 20 carbon atoms, dialkylamino of 2 to 40 carbon atoms, morpholino, piperidino, pyrrolidino, hydrogen, chloro, bromo, iodo, fluoro, perfluoroalkyl, perfluoroaryl, perfluoroaralkyl groups; 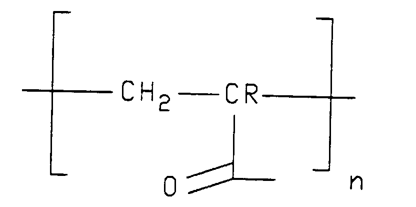
wherein
R is selected from the group consisting of hydrogen, alkyl, and aryl groups; and
n is at least 2; 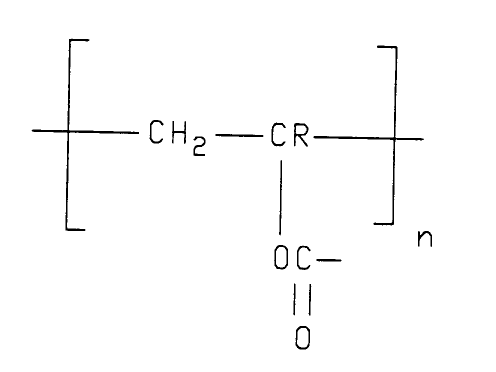
wherein
R is selected from the group consisting of hydrogen, alkyl, and aryl groups; and
n is at least 2;
and mixtures thereof.

5. The process for preparing an acid amide from a haloamine and an acid halide as recited in claim 4 wherein the haloamine is selected from the group consisting of monochloromelamine, dichloromelamine, trichloromelamine, tetrachloromelamine, pentachloromelamine, hexachloromelamine, haloacetoguanamines, halobenzoguanamines_{,} isomers thereof, and mixtures of any of the preceeding chloromelamines.

6. The process for preparing an acid amide from a haloamine and an acid halide as recited in claim 5 wherein the haloamine is N,N',N''-trichloromelamine or N,N,N',N',N'',N''-hexachloromelamine represented by the formulae:

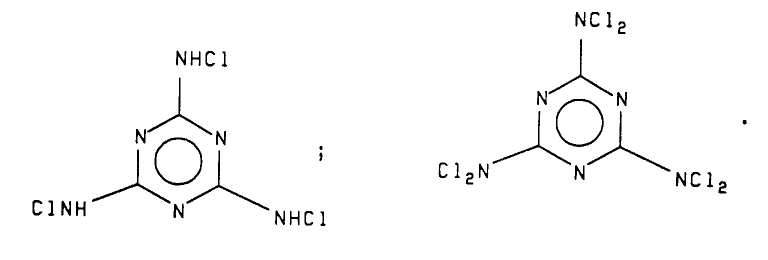

7. The process for preparing an acid amide from a haloamine and an acid halide as recited in claim 1 wherein the amine precursor of the haloamine has a solubility of less than 10 weight percent in halogenated organic solvents.

8. The process for preparing an acid amide from a haloamine and an acid halide as recited in claim 1 wherein the by-product is molecular halogen in the substantial absence of hydrogen halide.

9. The process for preparing an acid amide from a haloamine and an acid halide as recited in claim 1 wherein the substituents in the haloamine are connected to form a cyclic haloamine which is represented by the formula: 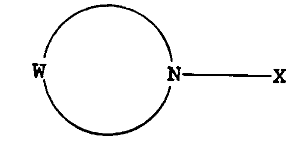 wherein W is a difunctional group capable of forming a ring at least three membered in size.

10. The process for preparing an acid amide from a haloamine and an acid halide as recited in claim 9 wherein the difunctional group W is selected from the group consisting of phthaloyl, succinoyl, maleoyl, alkylene, arylene, 2,2'-biphenylene, aralkylene, ortho-phenylenedisulfonyl, 1,2-ethylenedisulfonyl, 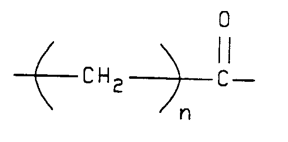 wherein n is at least 2, 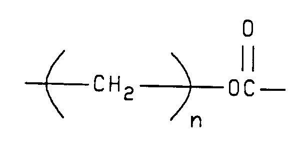 wherein n is at least 2, 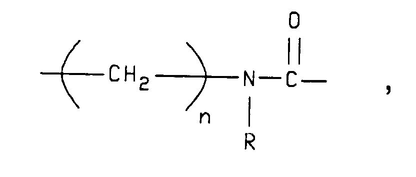 wherein R is selected from the group consisting of hydrogen, halogen, alkyl, aryl, and aralkyl groups, and
ortho-sulfobenzoyl group represented by the formula:

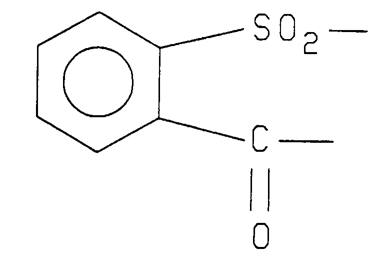

11. The process for preparing an acid amide from a haloamine and an acid halide as recited in claim 1 wherein the haloamine is a glycoluril derivative represented by the formula 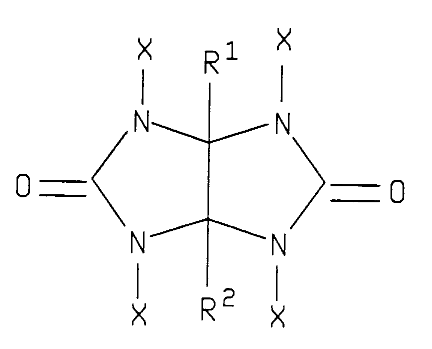 wherein
R¹ and R² are the same or different, and are selected from the group consisting of hydrogen, alkyl of 1 to 20 carbon atoms, aryl of 6 to 20 carbon atoms, and aralkyl of 7 to 20 carbon atoms;
and
wherein
X is a halogen selected from the group consisting of chloride, bromide, iodide, and fluroide.

12. The process for preparing an acid amide from a haloamine and an acid halide as recited in claim 11 wherein the haloamine is N,N',N'',N'',-tetrachloroglycoluril represented by the formula:

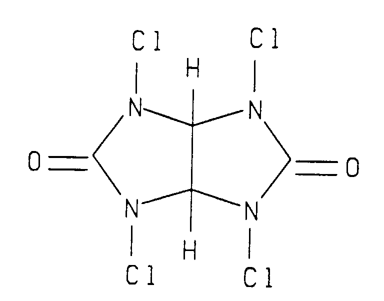

13. The process for preparing an acid amide from a haloamine and an acid halide as recited in claim 1 wherein the acid halide is represented by the formula:
Z(̵Y-X)_{M}
wherein
M is at least 1; and when M is at least 2, each (̵Y-X) group is the same or different;
X is a halogen selected from the group consisting of chloride, bromide, iodide, fluoride, and mixtures thereof;
Y in each (̵Y-X) group is the same or different and each is independently selected from the group consisting of the following functionalities represented by the formulae: 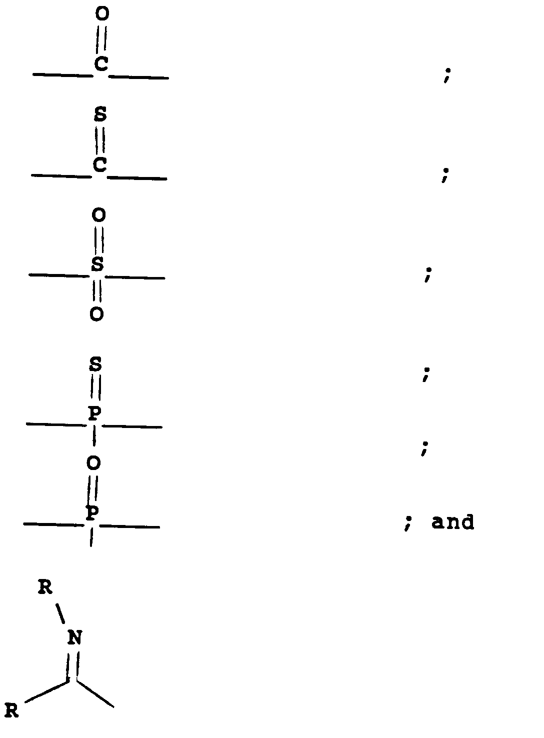
wherein each R is independently an alkyl, aryl, or an alkoxy group; and
Z is an M-functional anchor selected from the group consisting of monomeric or polymeric organic radical, hydrogen, chloro, bromo, iodo, fluoro, vinyl, alkyl of 1 to 20 carbon atoms, haloalkyl of 1 to 20 carbon atoms, aryl of 6 to 20 carbon atoms, haloaryl of 6 to 20 carbon atoms, aralkyl of 7 to 20 carbon atoms, haloaralkyl of 7 to 20 carbon atoms, acyl of 2 to 20 carbon atoms, haloacyl of 2 to 20 carbon atoms, halocarbonyl, alkoxycarbonyl of 2 to 20 carbon atoms, alkylthiocarbonyl of 2 to 20 carbon atoms, haloalkoxycarbonyl of 2 to 20 carbon atoms, aminocarbonyl, alkylaminocarbonyl of 2 to 20 carbon atoms, dialkylaminocarbonyl of 3 to 20 carbon atoms, alkenylaminocarbonyl of 4 to 20 carbon atoms, dialkenylaminocarbonyl of 7 to 40 carbon atoms, alkylalkenylaminocarbonyl of 5 to 20 carbon atoms, heteroaromatic ring, perfluoroalkyl of 1 to 20 carbon atoms, perfluoroaryl of 6 to 20 carbon atoms, perfluoroaralkyl of 7 to 20 carbon atoms, haloalkyl of 1 to 20 carbon atoms, alkoxycarbonylalkyl of 3 to 20 carbon atoms, cyanoalkyl of 2 to 20 carbon atoms, formylalkyl of 2 to 20 carbon atoms, ketoalkyl of 3 to 20 carbon atoms, trialkoxysilylalkyl of 4 to 20 carbon atoms, dialkylaminoalkyl of 3 to 20 carbon atoms, alkoxyalkyl of 2 to 20 carbon atoms, alkoxy of 1 to 20 carbon atoms, aryloxy of 6 to 20 carbon atoms, alkenyloxy of 3 to 20 carbon atoms, N,N-dialkylamino of 2 to 40 carbon atoms, N,N-dialkenylamino of 6 to 40 carbon atoms, N,N-diarylamino of 12 to 40 carbon atoms, N,N-alkylalkenylamino of 4 to 20 carbon atoms, N,N-alkylarylamino of 7 to 20 carbon atoms, N,N-alkenylarylamino of 9 to 20 carbon atoms, aziridino, azetidino, pyrrolidino, piperidino, morpholino, alkylmercapto of 1 to 20 carbon atoms, alkenylmercapto of 3 to 20 carbon atoms, and arylmercapto of 6 to 20 carbon atoms.

14. The process for preparing an acid amide from a haloamine and an acid halide as recited in claim 13 wherein the acid halide is selected from the group consisting of the following compounds represented by the formulae:

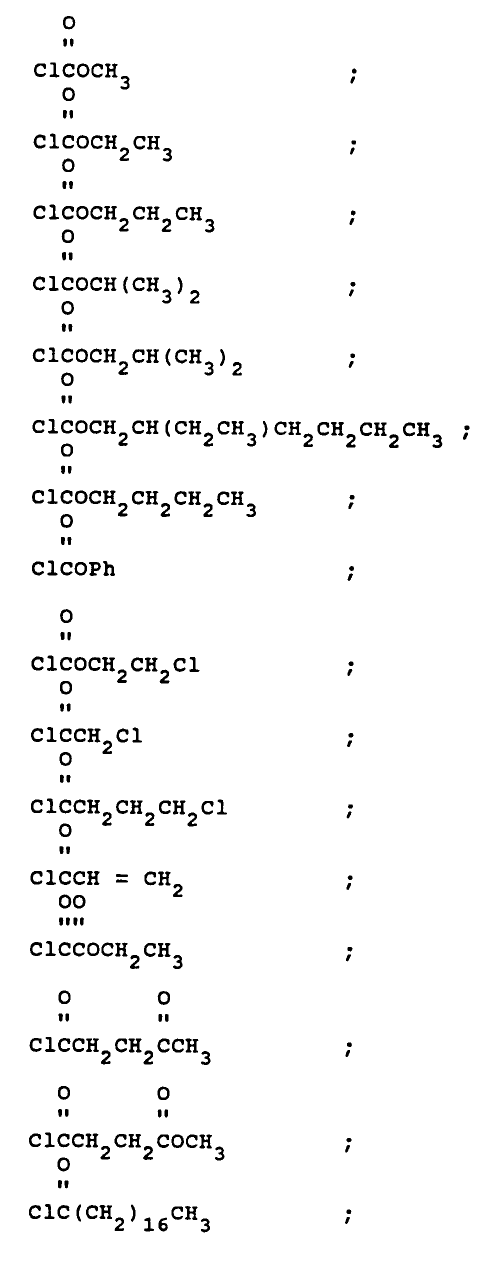

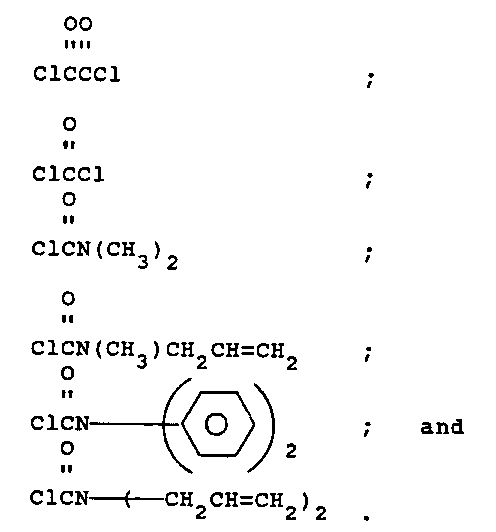

15. The process for preparing an acid amide from a haloamine and an acid halide as recited in claim 14 wherein the acid halide is selected from the group consisting of methylchloroformate, n-butyl chloroformate, phenyl chloroformate, 2-chloroethyl chloroformate, ethyl chloroformate, chloroacetyl chloride, 4-chlorobutyryl chloride, acryloyl chloride, methacryloyl chloride, oxalyl chloride, ethyl oxalyl chloride, levulinic chloride, methyl fumaroyl chloride, acetyl chloride, stearoyl chloride, and phosgene.

16. The process for preparing an acid amide from a haloamine and an acid halide as recited in claim 15 wherein the acid halide is selected from the group consisting of chloroacetyl chloride, 4-chlorobutyryl chloride, methyl chloroformate, normal butyl chloroformate, and phenyl chloroformate.

17. The process preparing an acid amide from a haloamine and an acid halide as recited in claim 16 wherein the haloamine is N,N',N''-trichloromelamine.

18. The process for preparing an acid amide from a haloamine and an acid halide as recited in claim 1 wherein the temperature is in the range of from -20^{o}C to 120^{o}C.

19. The process for preparing an acid amide from a haloamine and an acid halide as recited in claim 1 wherein the time is in the range of 10 minutes to 10 hours.

20. The process for preparing an acid amide from a haloamine and an acid halide as recited in claim 1 wherein the reaction is carried out in a liquid medium.

21. The process for preparing an acid amide from a haloamine and an acid halide as recited in claim 20 wherein the liquid medium is a solvent for said haloamine and said acid halide.

22. The process for preparing an acid amide from a haloamine and an acid halide as recited in claim 21 wherein the solvent is a halogenated solvent.

23. The process of claim 22 wherein the halogenated solvent is selected from the group consisting of methylene chloride, chloroform, carbon tetrachloride, 1,1,1-trichloroethane, chlorobenzene, ortho-dichloro-benzene, and mixtures thereof.

24. The process for preparing an acid amide from a haloamine and an acid halide as recited in claim 1 wherein the reaction mixture further comprises a catalyst selected from the group consisting of trialkyl phosphines, triaryl phosphines, quaternary ammonium halides, monomeric 4-dialkylaminopyridine, and a polymeric 4-dialkylamino pyridine derivative.

25. The process for preparing an acid amide from a haloamine and an acid halide as recited in claim 1 further comprising: isolating the reaction product by removing the volatiles under reduced pressure.

26. A composition of matter represented by the formula: 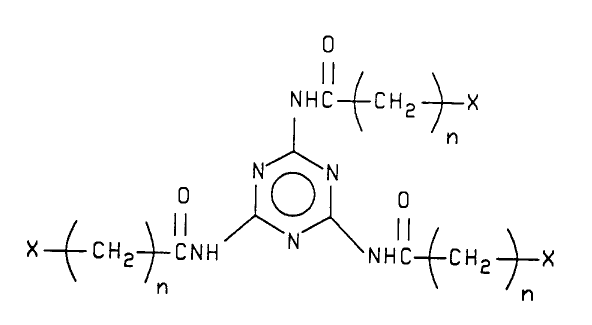 wherein
n is an integer from 1 to 5; and
X is a leaving group selected from the group consisting of chloride, bromide, iodide, fluoride, alkylsulfonate, arylsulfonate, and mixtures thereof.

27. The composition of matter as recited in claim 26 wherein n is 3, and X is chloride.

28. A process for producing a triazine tris-acid amide from melamine via trihalomelamine comprising:
(a) contacting said melamine with an halogen to produce hexahalomelamine;
(b) contacting melamine with hexahalomelamine to produce trihalomelamine; and
(c) contacting said trihalomelamine with an acid halide at a temperature and for a length of time sufficient to produce triazine tris-acid amide as a product and halogen as a by-product.

29. The process for producing a triazine tris-acid amide from melamine via trihalomelamine as recited in claim 28 wherein the halogen by-product in step (c) is recycled into step (a) to halogenate melamine to hexahalomelamine.

30. A process for producing a triazine tris-acid amide from melamine via hexahalomelamine comprising:
(I) contacting said melamine with a halogen to produce hexahalomelamine;
(II) contacting said hexahalomelamine with an acid halide at a temperature and for a length of time sufficient to produce triasine N,N',N''- trihalo tris- acid amide as an intermediate product and halogen as a by-product; and
(III)contacting the triazine N,N',N''-trihalo tris-acid amide intermediate of step (II) with hydrogen halide to produce triazine tris-acid amide as a final product and additional halogen as a by-product.

31. The process for producing a triazine tris-acid amide from melamine via hexahalomelamine as recited in claim 30 wherein the halogen by-product of steps (II) and (III) is recycled into step (I) to halogenate melamine to hexahalomelamine.

32. A composition of matter represented by the formula:

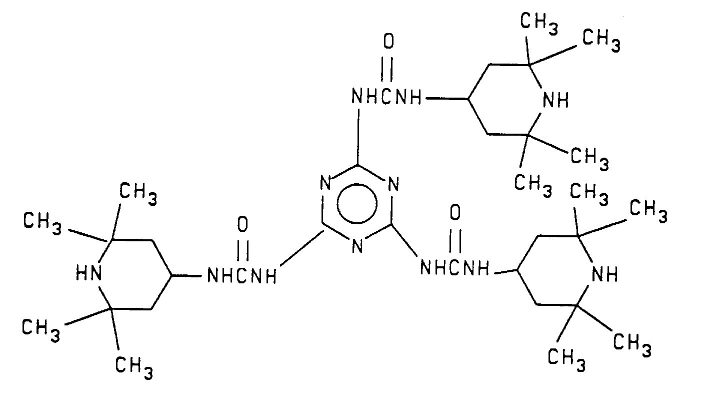

33. A composition of a matter represented by a formula: 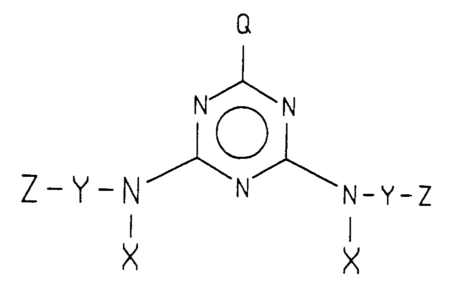 wherein
Q is 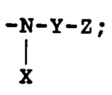 or
Q is selected from the group consisting of alkyl of 1 to 20 carbon atoms, alkenyl of 2 to 20 carbon atoms, aryl of 6 to 20 carbon atoms, aralkyl of 7 to 20 carbon atoms, alkoxy of 1 to 20 carbon atoms, aryloxy of 6 to 20 carbon atoms, alkylthio of 1 to 20 carbon atom, arylthio of 6 to 20 carbon atoms, alkylamino of 1 to 20 carbon atoms, dialkylamino of 2 to 40 carbon atoms, morpholino, piperidino, pyrrolidino, hydrogen, chloro, bromo, iodo, fluoro, perfluoroalkyl, perfluoroaryl, perfluoroaralkyl groups; and
wherein
X in each 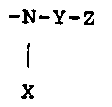 group is hydrogen or a halogen with the proviso that at least one X is a halogen independently selected from the group consisting of chloride, bromide, iodide, and fluoride; and
Y in each 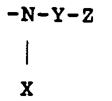 group is the same or different and each is independently selected from the group consisting of the following functionalities represented by the formulae: 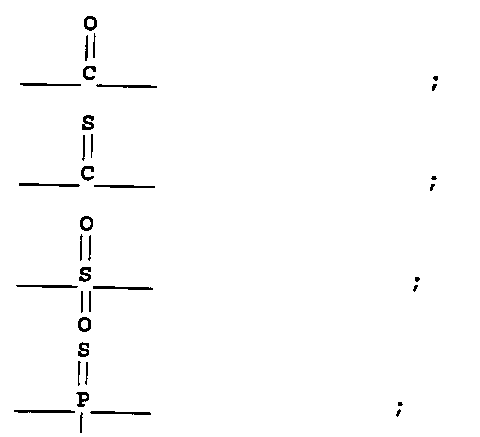 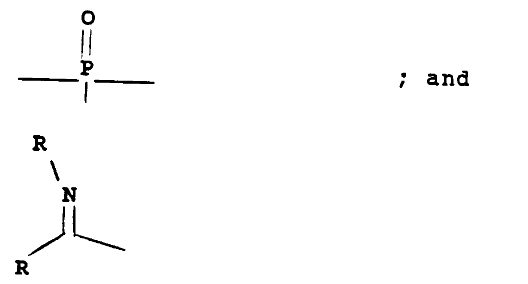 wherein each R is independently an alkyl, aryl, or an alkoxy group; and
Z is an M-functional anchor selected from the group consisting of monomeric or polymeric organic radical, hydrogen, chloro, bromo, iodo, fluoro, vinyl, alkyl of 1 to 20 carbon atoms, haloalkyl of 1 to 20 carbon atoms, aryl of 6 to 20 carbon atoms, haloaryl of 6 to 20 carbon atoms, aralkyl of 7 to 20 carbon atoms, haloaralkyl of 7 to 20 carbon atoms, acyl of 2 to 20 carbon atoms, haloacyl of 2 to 20 carbon atoms, halocarbonyl, alkoxycarbonyl of 2 to 20 carbon atoms, alkylthiocarbonyl of 2 to 20 carbon atoms, haloalkoxycarbonyl of 2 to 20 carbon atoms, aminocarbonyl, alkylaminocarbonyl of 2 to 20 carbon atoms, dialkylaminocarbonyl of 3 to 20 carbon atoms, alkenylaminocarbonyl of 4 to 20 carbon atoms, dialkenylaminocarbonyl of 7 to 40 carbon atoms, alkylalkenylaminocarbonyl of 5 to 20 carbon atoms, heteroaromatic ring, perfluoroalkyl of 1 to 20 carbon atoms, perfluoroaryl of 6 to 20 carbon atoms, perfluoro aralkyl of 7 to 20 carbon atoms, haloalkyl of 1 to 20 carbon atoms, alkoxycarbonylalkyl of 3 to 20 carbon atoms, cyanoalkyl of 2 to 20 carbon atoms, formylalkyl of 2 to 20 carbon atoms, ketoalkyl of 3 to 20 carbon atoms, trialkoxysilylalkyl of 4 to 20 carbon atoms, dialkylaminoalkyl of 3 to 20 carbon atoms, alkoxyalkyl of 2 to 20 carbon atoms, alkoxy of 1 to 20 carbon atoms, aryloxy of 6 to 20 carbon atoms, alkenyloxy of 3 to 20 carbon atoms, N,N-dialkylamino of 2 to 40 carbon atoms, N,N-dialkenylamino of 6 to 40 carbon atoms, N,N-diarylamino of 12 to 40 carbon atoms, N,N-alkylalkenylamino of 4 to 20 carbon atoms, N,N-alkylarylamino of 7 to 20 carbon atoms, N,N-alkenylarylamino of 9 to 20 carbon atoms, aziridino, azetidino, pyrrolidino, piperidino, morpholino, alkylmercapto of 1 to 20 carbon atoms, alkenylmercapto of 3 to 20 carbon atoms, and arylmercapto of 6 to 20 carbon atoms.

34. The compound N,N',N''-tri-paranitrobenzolmelamine.

35. The compound triazine tris-(2-chloroethylcarbamate).
